# EUROPEAN PATENT APPLICATION

(11) **EP 4 722 354 A1**
(43) Date of publication of application: **08.04.2026**
(21) Application number: 24815645.7
(22) Date of filing: 02.04.2024
(51) Int. Cl.: C12N 9/00, C12N 15/11, C12N 15/52

(54) **SINGLE-STRANDED DNA LINKING COMPOSITION**

(30) Priority: 30.05.2023 JP 2023088967
(71) Applicant: Kyushu University, National University Corporation, Nishi-ku Fukuoka-shi Fukuoka 819-0395 (JP)
(72) Inventor: MIURA, Fumihito, Fukuoka-shi, Fukuoka 819-0395 (JP)
(74) Representative: J A Kemp LLP
(86) International application number: PCT/JP2024/080045
(87) International publication number: WO 2024/248166

(57) **Abstract**

Provided is, for example, a novel enzyme composition having efficient single-stranded DNA ligation activity. The present invention relates to, for example, a composition for ligating single-stranded DNA, used for ligating at least one single-stranded DNA as an acceptor to at least one single-stranded DNA as a donor, the composition comprising (a) a protein comprising an amino acid sequence represented by SEQ ID NO: 2 or 4, and/or (b) a protein which comprises an amino acid sequence having 70% or higher identity to the amino acid sequence represented by SEQ ID NO: 2 or 4 and has single-stranded DNA ligation activity.

## Description

### Technical Field

The present invention relates to, a composition for ligating single-stranded DNA, a single-stranded DNA ligation agent, a kit for ligating single-stranded DNA, and others which are used for ligating single-stranded DNA as an acceptor to single-stranded DNA as a donor.

### Background Art

Although many applications are expected to be realized with an enzyme having strong single-stranded DNA (ssDNA) ligation activity, no such enzyme is currently available. A large demand for a single-stranded DNA ligation technique is readily understandable in view of sequencing library preparation. Many kinds of single-stranded DNA have garnered interest recently, and their efficient sequencing has been demanded. For example, short single-stranded DNA in the cell-free fraction of blood has been found to have characteristics different from those of nucleosome-sized cell-free DNA (cfDNA), and analysis thereon is expected (see Non Patent Literatures 1 to 3). DNA extracted from bones excavated from remains dating back several thousand years is generally heavily degraded, damaged, and single-stranded (see Non Patent Literature 4). A lot of potential single-stranded DNAs require sequencing. However, most commercially available library preparation kits are currently adapted for only double-stranded DNA, and a limited number of kits are available for single-stranded DNA. These kits and protocols adapted for single-stranded DNA still do not have sufficiently high library preparation efficiency (see Non Patent Literatures 1, 5, and 6).

To break through the lack of an approach of ligating such single-stranded DNAs with high efficiency, the present inventor has recently developed a technique designated as a TACS ligation method (see Non Patent Literature 7). In this method, the 3' end of target single-stranded DNA is first tailed with a few adenylic acids using terminal deoxyribonucleotidyl transferase (TdT) (see Non Patent Literature 8). This short RNA stretch (ribotail) is used as a perfect substrate for RNA ligase and serves as a connector for enhancing its ligation activity (see Non Patent Literature 9). As a result, the single-stranded DNA ligation activity of the RNA ligase in the presence of TdT exceeds 80% under optimum conditions (see Non Patent Literature 7). The principles of this TACS ligation can be used for highly efficient sequencing library preparation from cell-free DNA (see Non Patent Literature 1) and degraded DNA extracted from ancient human bones owing to the high single-stranded DNA ligation activity. However, the ribotail, which is introduced in this course, has limitations in such a way that the ribotail cannot be used for developing technologies in which the insertion of base or the presence of RNA is disadvantageous.

### Citation List

### Non Patent Literature

[Non Patent Literature 1] Hisano, O., Ito, T. and Miura, F. (2021) Short single-stranded DNAs with putative non-canonical structures comprise a new class of plasma cell-free DNA. BMC Biol, 19, 225.
[Non Patent Literature 2] Cheng, J., Morselli, M., Huang, W.-L., Heo, Y.J., Pinheiro-Ferreira, T., Li, F., Wei, F., Chia, D., Kim, Y., He, H.-J. et al. (2022) Plasma contains ultrashort single-stranded DNA in addition to nucleosomal cell-free DNA. iScience, 25, 104554.
[Non Patent Literature 3] Hudecova, I., Smith, C.G., Hänsel-Hertsch, R., Chilamakuri, C.S., Morris, J.A., Vijayaraghavan, A., Heider, K., Chandrananda, D., Cooper, W.N., Gale, D. et al. (2022) Characteristics, origin, and potential for cancer diagnostics of ultrashort plasma cell-free DNA. Genome research, 32, 215-227.
[Non Patent Literature 4] Meyer, M., Kircher, M., Gansauge, M.T., Li, H., Racimo, F., Mallick, S., Schraiber, J.G., Jay, F., Prufer, K., de Filippo, C. et al. (2012) A high-coverage genome sequence from an archaic Denisovan individual. Science, 338, 222-226.
[Non Patent Literature 5] Gansauge, M.T. and Meyer, M. (2013) Single-stranded DNA library preparation for the sequencing of ancient or damaged DNA. Nature protocols, 8(4), 737-748.
[Non Patent Literature 6] Gansauge, M.T., Gerber, T., Glocke, I., Korlevic, P., Lippik, L., Nagel, S., Riehl, L.M., Schmidt, A. and Meyer, M. (2017) Single-stranded DNA library preparation from highly degraded DNA using T4 DNA ligase. Nucleic acids research, 45(10), e79.
[Non Patent Literature 7] Miura, F., Shibata, Y., Miura, M., Sangatsuda, Y., Hisano, O., Araki, H. and Ito, T. (2019) Highly efficient single-stranded DNA ligation technique improves low-input whole-genome bisulfite sequencing by post-bisulfite adaptor tagging. Nucleic acids research, 47(15), e85.
[Non Patent Literature 8] Schmidt, W.M. and Mueller, M.W. (1996) Controlled ribonucleotide tailing of cDNA ends (CRTC) by terminal deoxynucleotidyl transferase: a new approach in PCR-mediated analysis of mRNA sequences. Nucleic acids research, 24, 1789-1791.
[Non Patent Literature 9] Blondal, T., Thorisdottir, A., Unnsteinsdottir, U., Hjorleifsdottir, S., Aevarsson, A., Ernstsson, S., Fridjonsson, O.H., Skirnisdottir, S., Wheat, J.O., Hermannsdottir, A.G. et al. (2005) Isolation and characterization of a thermostable RNA ligase 1 from a Thermus scotoductus bacteriophage TS2126 with good single-stranded DNA ligation properties. Nucleic acids research, 33, 135-142.
[Non Patent Literature 10] Zhang, X.H. and Chiang, V.L. (1996) Single-stranded DNA ligation by T4 RNA ligase for PCR cloning of 5'-noncoding fragments and coding sequence of a specific gene. Nucleic acids research, 24, 990-991.
[Non Patent Literature 11] Tessier, D.C., Brousseau, R. and Vernet, T. (1986) Ligation of single-stranded oligodeoxyribonucleotides by T4 RNA ligase. Anal Biochem, 158, 171-178.
[Non Patent Literature 12] Blondal, T., Hjorleifsdottir, S.H., Fridjonsson, O.F., Aevarsson, A., Skirnisdottir, S., Hermannsdottir, A.G., Hreggvidsson, G.O., Smith, A.V. and Kristjansson, J.K. (2003) Discovery and characterization of a thermostable bacteriophage RNA ligase homologous to T4 RNA ligase 1. Nucleic acids research, 31, 7247-7254.
[Non Patent Literature 13] Zhelkovsky, A.M. and McReynolds, L.A. (2012) Structure-function analysis of Methanobacterium thermoautotrophicum RNA ligase - engineering a thermostable ATP independent enzyme. BMC Mol Biol, 13, 24.
[Non Patent Literature 14] Miura F. et al., Nucleic Acids Res. 2012, 40, e136
[Non Patent Literature 15] Miura F, Kawaguchi N, Yoshida M, Uematsu C, Kito K, Sakaki Y, Ito T. Absolute quantification of the budding yeast transcriptome by means of competitive PCR between genomic and complementary DNAs. BMC Genomics. 2008;9:574.
[Non Patent Literature 16] Miura F, et al., A highly efficient scheme for library preparation from single-stranded DNA, Scientific Reports, 13, 13913, 2023

### Summary of Invention

Under these circumstances, there has been a demand for the development of a novel enzyme composition or the like having efficient single-stranded DNA ligation activity without the addition of ribotail.

The present invention has been made in light of these circumstances and provides the following composition for ligating single-stranded DNA, a single-stranded DNA ligation agent, a kir for ligating single-stranded DNA, and others.
[1] A composition for ligating single-stranded DNA, used for ligating at least one single-stranded DNA as an acceptor to at least one single-stranded DNA as a donor, the composition comprising
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity.
[2] The composition according to [1], wherein the acceptor DNA is 12 or more bases long, and/or the donor DNA is 10 or more bases long.
[3] The composition according to [1], further comprising PEG.
[4] A single-stranded DNA ligation agent, used for ligating at least one single-stranded DNA as an acceptor to at least one single-stranded DNA as a donor, the ligation agent comprising
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity.
[5] The ligation agent according to [4], wherein the acceptor DNA is 12 or more bases long, and/or the donor DNA is 10 or more bases long.
[6] The ligation agent according to [4], further comprising PEG.
[7] A kir for ligating single-stranded DNA, used for ligating at least one single-stranded DNA as an acceptor to at least one single-stranded DNA as a donor, the kit comprising
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity.
[8] The kit according to [7], wherein the acceptor DNA is 12 or more bases long, and/or the donor DNA is 10 or more bases long.
[9] The kit according to [7], further comprising PEG.
[10] A method for producing ligated single-stranded DNA, comprising bringing at least one single-stranded DNA as an acceptor and at least one single-stranded DNA as a donor into contact with
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity,
   to ligate the acceptor DNA to the donor DNA.
[11] A method for ligating single-stranded DNA, comprising bringing at least one single-stranded DNA as an acceptor and at least one single-stranded DNA as a donor into contact with
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity,
   to ligate the acceptor DNA to the donor DNA.
[12] The method according to [10] or [11], wherein the acceptor DNA is 12 or more bases long, and/or the donor DNA is 10 or more bases long.
[13] Use of
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity
   in the method according to [10] or [11].
[14]
   (a) A protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity
   for use in the method according to [10] or [11].
[15] A method for preparing a double-stranded DNA library from a single-stranded DNA fragment, the preparation method comprising the steps of:
   (A) ligating a 5'-terminally phosphorylated single-stranded adapter sequence to the target single-stranded DNA fragment;
   (B) performing DNA replication beginning at the adapter of the ligated sequence to form double-stranded DNA;
   (C) ligating a topoisomerase-complexed double-stranded adapter sequence to ends tailed with no adapter sequence of the double-stranded DNA; and
   (D) using the ligated double-stranded DNA as a template in PCR to obtain a library comprising a plurality of the double-stranded DNA, wherein
   the ligation in the step (A) is performed by a ligation method using
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity.
[16] A method for preparing a double-stranded DNA library from a single-stranded DNA fragment, the preparation method comprising the steps of:
   (i) ligating a 5'-terminally phosphorylated single-stranded adapter sequence A1 to the 3' end of a single-stranded DNA fragment dephosphorylated at both ends to form adapter-tailed (adapter-tagged) single-stranded DNA;
   (ii) annealing a single-stranded adapter sequence A2 to the adapter sequence A1 moiety contained in the adapter-tailed single-stranded DNA, followed by extension from the 3' end of the adapter sequence A2 with DNA polymerase to form adapter-tailed double-stranded DNA;
   (iii) reacting a double-stranded adapter sequence B1 with topoisomerase to form a topoisomerase-complexed double-stranded adapter sequence B2, wherein the double-stranded adapter sequence B1 is phosphorylated at only one 5' end and has a topoisomerase recognition sequence in a region containing a 3' end complementary to this phosphorylated 5' end, and wherein the topoisomerase-complexed double-stranded adapter sequence B2 has the topoisomerase conjugated with the 3' end of the topoisomerase recognition sequence;
   (iv) ligating the topoisomerase-complexed double-stranded adapter sequence B2 to ends tailed with no adapter sequence of the adapter-tailed double-stranded DNA to form both-terminally adapter-tailed double-stranded DNA; and
   (v) using the both-terminally adapter-tailed double-stranded DNA as a template in PCR to obtain a library comprising a plurality of the double-stranded DNA, wherein
   the ligation and the formation in the step (i) are performed by a ligation method using
   (a) a protein comprising an amino acid sequence represented by SEQ ID NO: 2 or 4, and/or
   (b) a protein which comprises an amino acid sequence having 70% or higher identity to the amino acid sequence represented by SEQ ID NO: 2 or 4 and has single-stranded DNA ligation activity.

In this context, in the preparation method according to [16],
it is preferred that the topoisomerase-complexed double-stranded adapter sequence B2 should not be ligated to a double-stranded adapter sequence C formed by the annealing of an unreacted form of the single-stranded adapter sequence A1 to an unreacted form of the single-stranded adapter sequence A2.

It is also preferred that the double-stranded adapter sequence B1 should have a nick in a region containing the phosphorylated 5' end, and the single-stranded adapter sequence A1 should have uracil or dSpacer in the sequence.

The step (iv) preferably comprises adding endonuclease to the reaction system after the formation.

[17] A double-stranded DNA library obtainable by a preparation method according to [15] or [16].

### Advantageous Effects of Invention

The present invention can provide a composition for ligating single-stranded DNA as a novel enzyme composition having efficient single-stranded DNA ligation activity, a single-stranded DNA ligation agent, a kir for ligating single-stranded DNA, and others. Use of the composition for ligating single-stranded DNA or the other according to the present invention enables single-stranded DNAs as a donor and an acceptor to be ligated without ribotailing and can achieve highly efficient sequencing library preparation, for example. In this respect, the composition for ligating single-stranded DNA, and others according to the present invention is useful.

### Brief Description of Drawings

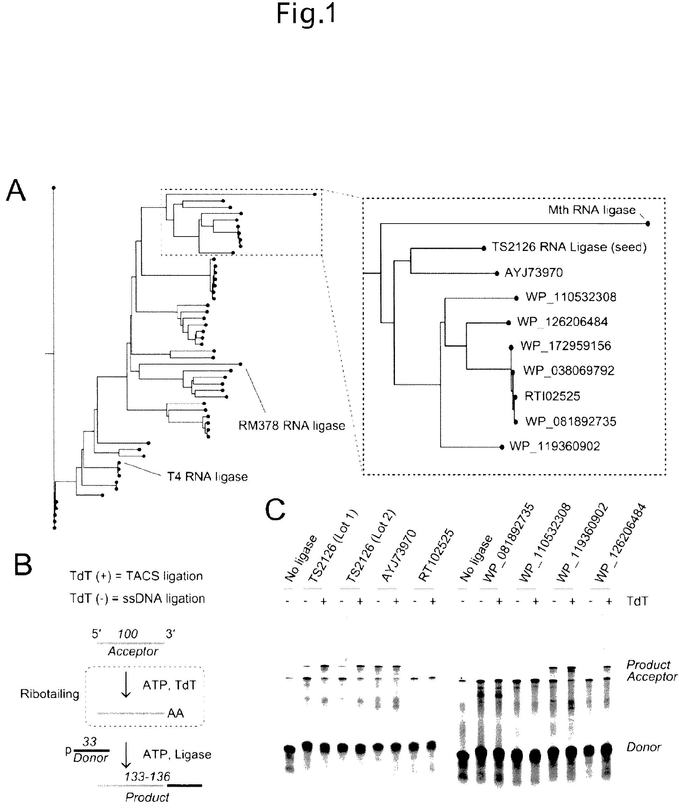

[Figure 1] Figure 1 is a diagram regarding the identification of single-stranded DNA ligase.
   A. Phylogenetic tree of proteins homologous to TS2126 RNA ligase. The view in the dashed box is enlarged in the right panel.
   B. The assay design used. TdT in reaction attaches a few adenylic acids to the 3' end of an acceptor. This RNA enhances the ligation activity of RNA ligase. This is so-called TACS ligation. Highly efficient ligation reaction in the presence of TdT was confirmed in some cases. The occurrence of ligation in the absence of TdT means that the enzyme has genuine single-stranded DNA ligation activity.
   C. Gel images obtained by actual screening. Successful ligation is indicated by an upward shift of the band.

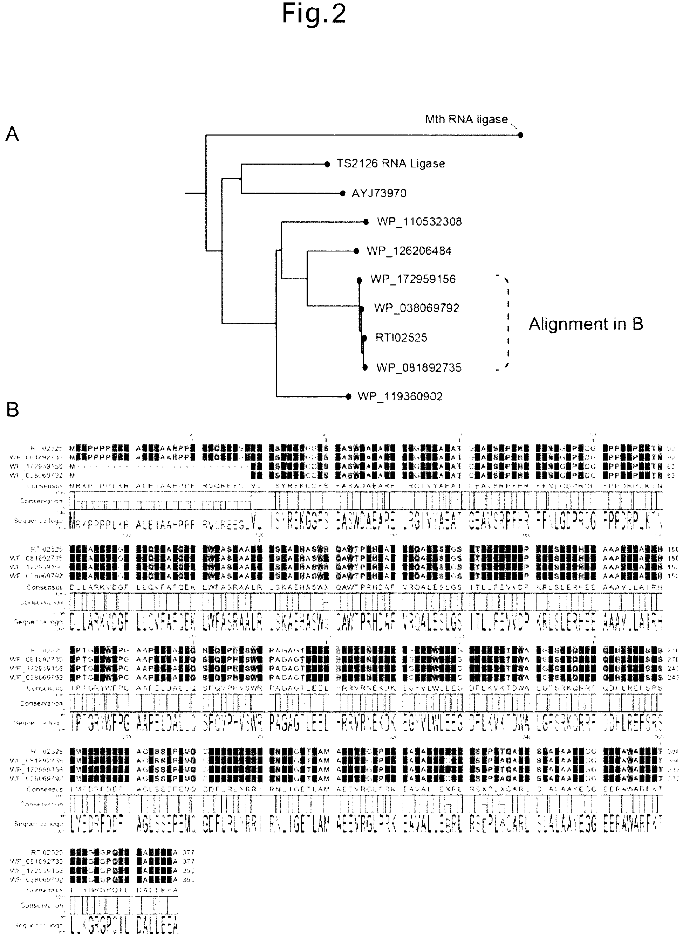

[Figure 2] Figure 2 is a diagram showing phylogenetic relationship among candidate proteins.
   A. Partial phylogenetic tree. Homologies of the amino acid sequences of the proteins homologous to TS2126 RNA ligase. The same as the enlarged portion of Figure 1A is indicated.
   B. Multiple alignment of the amino acid sequences shown in Figure 2A. WP_172959156 and RTI02525 have totally the same amino acid sequence except for N-terminal extension. The same holds true for WP_081892735 and WP_038069792.

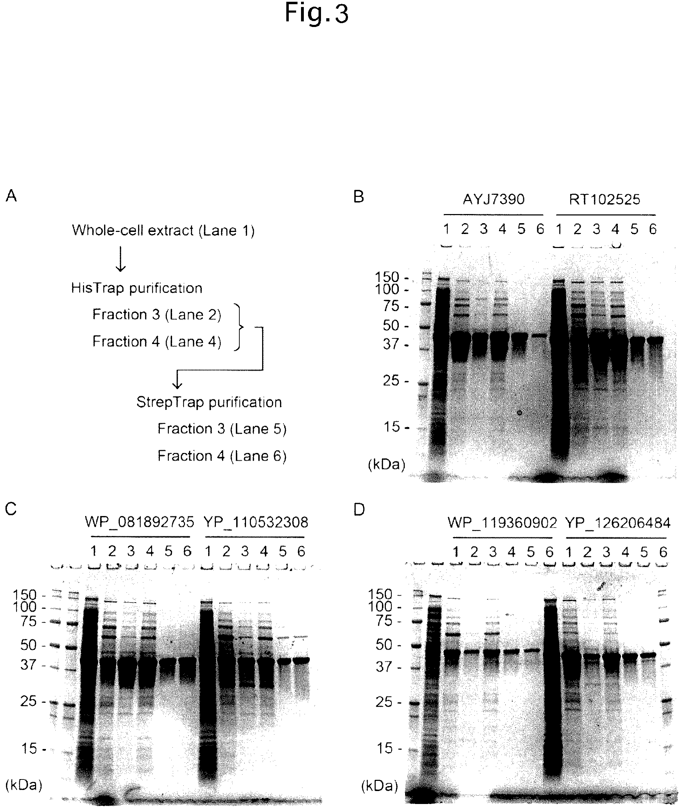

[Figure 3] Figure 3 is a diagram showing dual affinity purification of six candidate proteins.
   A. Purification strategy
   B to D. Results of analyzing purified proteins by SDS-PAGE. The lane numbers are as defined in Figure 3A. Unpurified lysates and the purified proteins were analyzed with anyKd Mini-PROTEAN TGX Precast Gel (Bio-Rad Laboratories, Inc., Hercules, CA). After electrophoresis, the gel was stained with Bullet CBB Stain One (Nacalai Tesque Inc., Kyoto, Japan) and photographed with ChemiDoc Touch Imaging System (Bio-Rad Laboratories, Inc.).

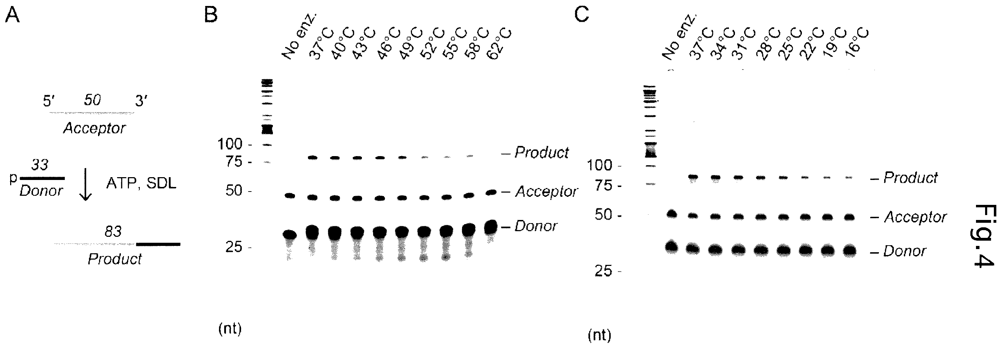

[Figure 4] Figure 4 is a diagram showing the optimum reaction temperature of AYJ73970.
   A. Assay design. The italic numbers depict the lengths of oligonucleotides.
   B and C. Analysis images obtained by denaturing gel electrophoresis after reaction. 25 µL of a reaction solution containing 50 mM HEPES-KOH, pH 7.5, 10 mM MgCl₂, 0.05% (v/v) Tritton X-100, 400 µM ATP, 16%(w/v) PEG6000, 5 pmol acceptor DNA (N50, see Table 1), 50 pmol donor DNA (P-anti-PEA2-thio5-P, see Table 1), and 3 µg of SDL (AYJ73970) was provided and incubated at each temperature for 1 hour. A lane involving no ligase was provided as a control. 20 µL of Buffer B2 (Qiagen N.V., Hilden, Germany) and 5 µL of proteinase K (Qiagen N.V.) were added to the solution thus reacted, and the resultant was incubated at 50°C for 15 minutes. Subsequently, 2 µL of Sera-Mag Carboxylate Beads (Cytiva, Marlborough, MA) and 50 µL of ethanol were added to the reaction product and the resultant was incubated at room temperature for 5 minutes. After the beads were rinsed with 200 µL of 70% ethanol, the purified DNA was eluted with 10 mM Tris-Acetate, pH 8.0. The purified DNA was mixed with an equal volume of formamide, heated at 70°C for 5 minutes, and loaded to 10% Novex TBE-Urea Gel (Thermo Fisher Scientific Inc., Waltham, MA). After electrophoresis, the gel was stained with SYBR Gold Gel Stain (Thermo Fisher Scientific Inc.) and photographed with ChemiDoc Touch Imaging System.
   In Figure 4 or later (Figures 4 to 14) of the present application, the term "SDL" means ligase derived from Thermus phage phiLo (Tph ssDNA ligase, GenBank Accession No: AYJ73970) and is also simply referred to as "AYJ73970".
[Figure 5] Figure 5 is a diagram showing the influence of polyethylene glycol (PEG) with varying molecular weights.
   A. Assay design.
   B to D. Denaturing gel electrophoresis images of reaction products. 25 µL of a reaction solution containing 50 mM Tris-HCl, pH 8.5, 5 mM MgCl₂, 0.05% (v/v) Tritton X-100, 400 µM ATP, 5 pmol acceptor DNA (N50), 50 pmol donor DNA (P-anti-PEA2-thio5-P, see Table 1), 3 µg of SDL, and each PEG with a designated concentration was provided and incubated at 37°C for 1 hour. A reaction solution containing no ligase was provided as a control. An approach of analyzing reaction results is the same as in Figure 4.

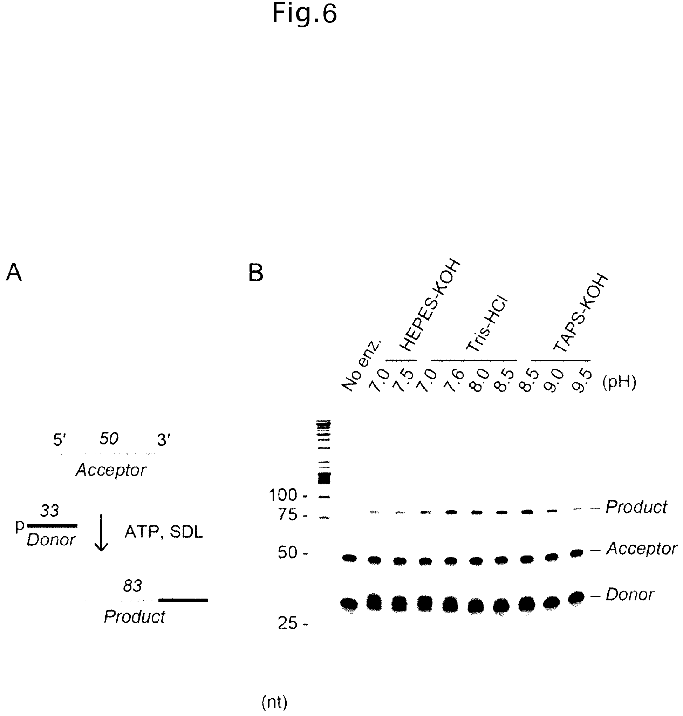

[Figure 6] Figure 6 is a diagram showing the optimization of reaction pH and a buffering system.
   A. Assay design
   B. Denaturing gel electrophoresis image of reaction products. 25 µL of a reaction solution containing 50 mM designated buffering component, 5 mM MgCl₂, 0.05% (v/v) Tritton X-100, 400 µM ATP, 16% (w/v) PEG6000, 5 pmol acceptor DNA (N50), 50 pmol donor DNA (P-anti-PEA2-thio5-P, see Table 1), and 3 µg of SDL was prepared and incubated at 37°C for 1 hour. A reaction solution containing no ligase was provided as a control. An approach of analyzing reaction results is the same as in Figure 4.

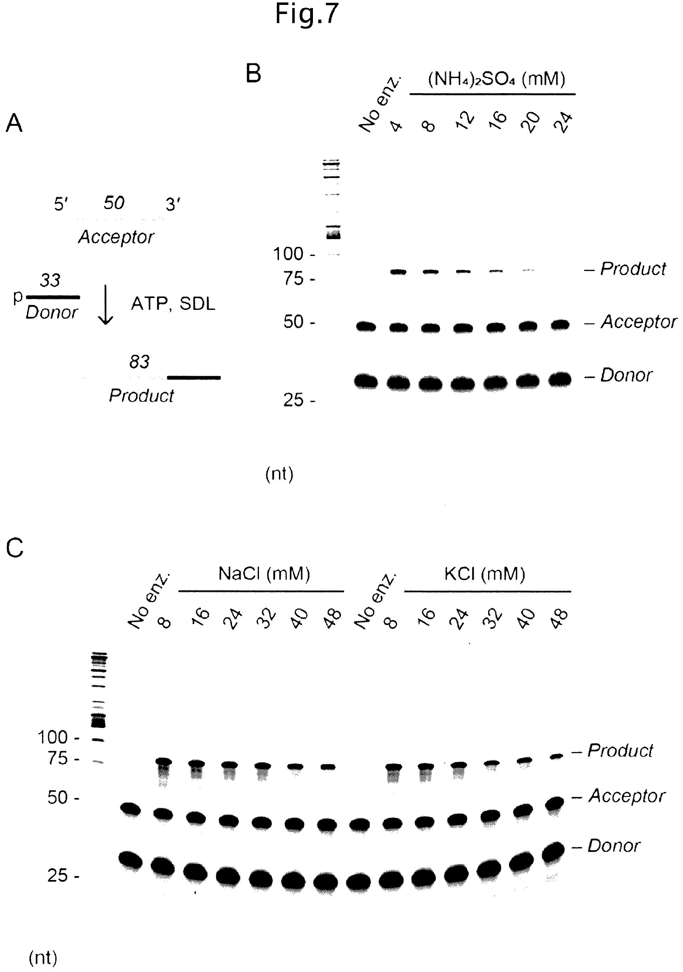

[Figure 7] Figure 7 is a diagram showing the optimum ion concentration of ssDNA ligase.
   A. Assay design.
   B and C. Denaturing gel electrophoresis images of reaction products. 25 µL of a reaction solution containing a designated ion as well as 50 mM Tris-HCl, pH 8.5, 5 mM MgCl₂, 0.05% (v/v) Tritton X-100, 400 µM ATP, 16% (w/v) PEG6000, 5 pmol acceptor DNA (N50), 50 pmol donor DNA (P-anti-PEA2-thio5-P, see Table 1), and 3 µg of SDL was prepared and incubated at 37°C for 1 hour. An approach of analyzing reaction results is the same as in Figure 4.

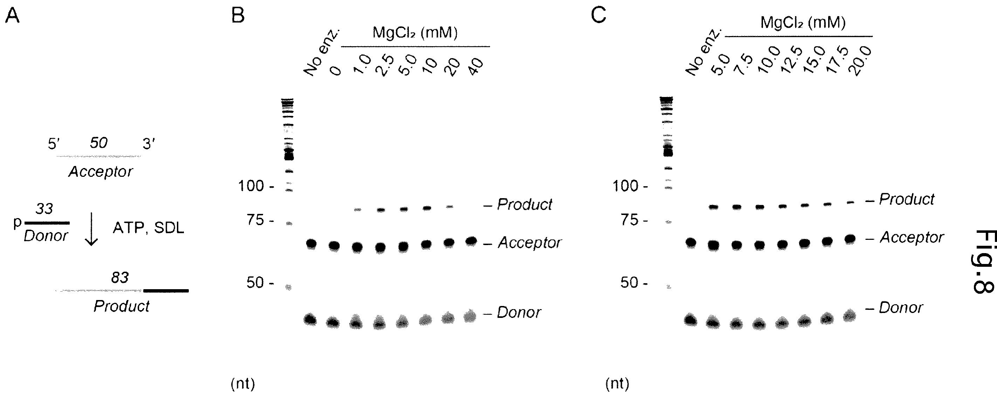

[Figure 8] Figure 8 is a diagram showing the optimum magnesium chloride (MgCl₂) concentration of ssDNA ligase.
   A. Assay design.
   B and C. Denaturing gel electrophoresis images of reaction products. 25 µL of a reaction solution containing a designated magnesium concentration as well as 50 mM Tris-HCl, pH 8.5, 0.05% (v/v) Tritton X-100, 400 µM ATP, 16% (w/v) PEG6000, 5 pmol acceptor DNA (N50), 50 pmol donor DNA (P-anti-PEA2-thio5-P, see Table 1), and 3 µg of SDL was prepared and incubated at 37°C for 1 hour. An approach of analyzing reaction results is the same as in Figure 4.

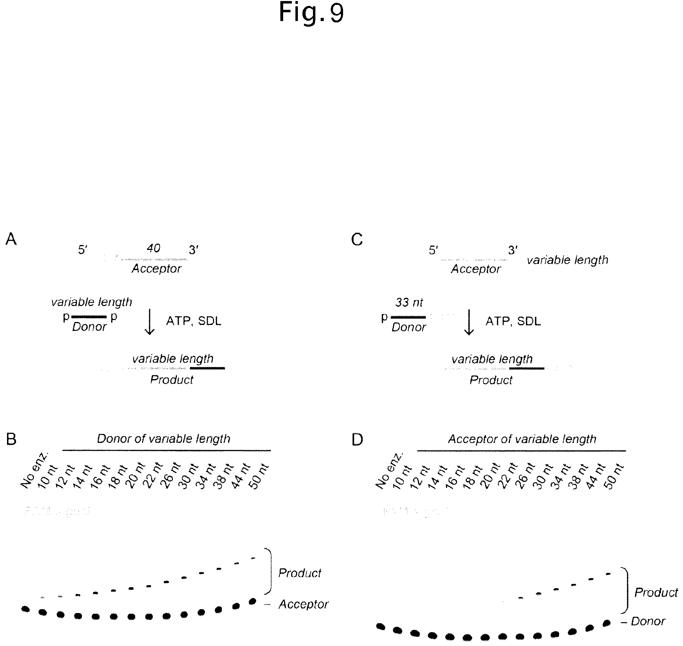

[Figure 9] Figure 9 is a diagram showing study on the smallest sizes of a donor and an acceptor of ssDNA ligase.
   A and C. Assay designs for studying nucleotide lengths necessary for the donor (A) and the acceptor (C).
   B and D. Denaturing gel electrophoresis images of reaction products. 25 µL of a reaction solution containing 50 mM Tris-HCl, pH 8.5, 10 mM MgCl₂, 0.05% (v/v) Tritton X-100, 400 µM ATP, 32% (v/v) PEG600, oligonucleotides (mentioned later), and 3 µg of SDL was prepared and incubated at 37°C for 1 hour. The reaction product was diluted with formamide at a ratio of 1:9, thermally denatured at 95°C for 3 minutes, and loaded to 10% Novex TBE-Urea Gel. After electrophoresis, fluorescent signals of FAM were photographed with ChemiDoc Touch Imaging System without staining. 50 pmol acceptor DNA (FAM-N40, see Table 1) and 100 pmol donor DNA (chemically synthesized product of a mixed sequence of four types of bases) were used in the assay to examine the length of the donor. 50 pmol donor DNA (P-anti-PEA2-thio5-FAM, see Table 1) and 100 pmol acceptor DNA (chemically synthesized product of a mixed sequence of four types of bases, both the 5' end and the 3' end of which were modified by phosphorylation) were used in the study on the length of the acceptor.

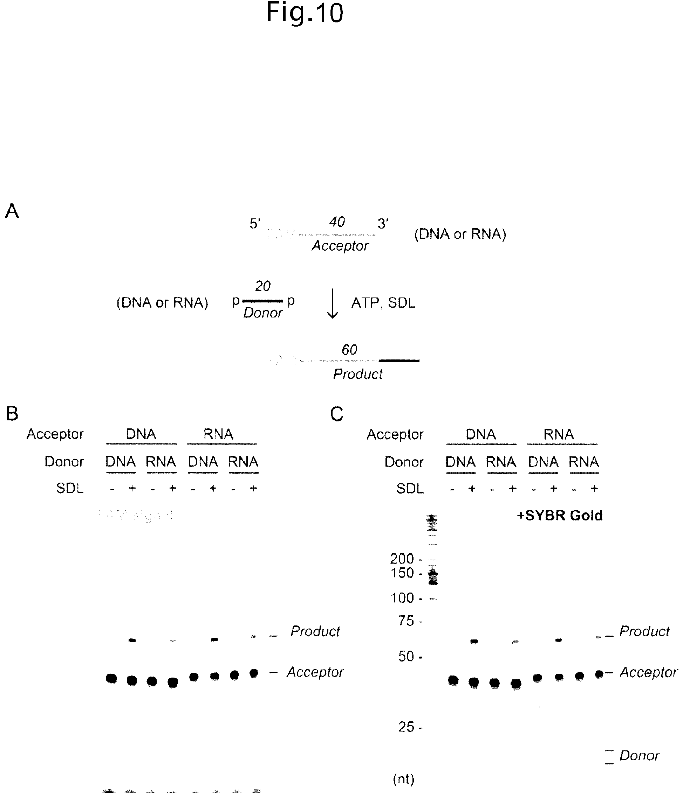

[Figure 10] Figure 10 is a diagram showing the reactivity of ssDNA ligase with RNA.
   A. Assay design.
   B and C. Denaturing gel electrophoresis images of reaction products. 25 µL of a reaction solution containing 50 mM Tris-HCl, pH 8.5, 10 mM MgCl₂, 0.05% (v/v) Tritton X-100, 400 µM ATP, 32% (v/v) PEG600, a donor (DNA or RNA, FAM-N40 or FAM-rN40, see Table 1), an acceptor (DNA or RNA, N20 or rN20, see Table 1), and 3 µg of SDL was provided and incubated at 37°C for 1 hour. The reaction solution (1 µL) was mixed with 10 µL of formamide, and the resultant was incubated at 70°C for 5 minutes, and loaded to 10% Novex TBE-Urea Gel. After electrophoresis, fluorescent signals of FAM were photographed with ChemiDoc Touch Imaging System without staining (B). Then, the gel was stained with SYBR Gold and photographed again (C).

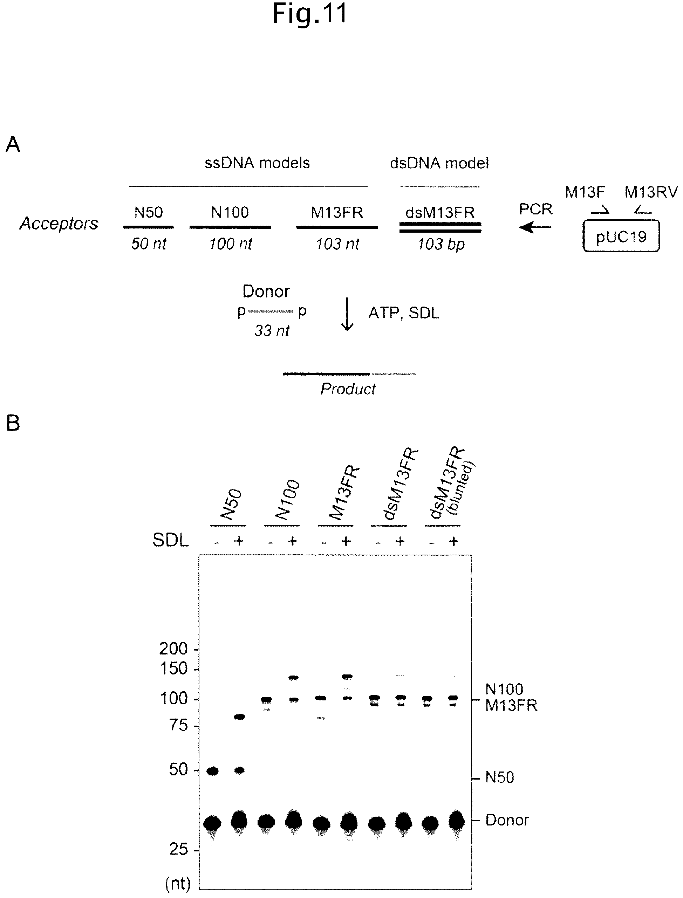

[Figure 11] Figure 11 is a diagram showing the ligation activity of SDL against ssDNA and dsDNA.
   A. Assay design. dsDNA was prepared by PCR. Half the amount of the PCR products was used directly, and the remaining amount of the PCR products was blunt-ended by the addition of Klenow fragment. The other models of single-stranded acceptor DNA were provided by chemical synthesis.
   B. Denaturing gel electrophoresis image of reaction products. 25 µL of a reaction solution containing 50 mM Tris-HCl, pH 8.5, 10 mM MgCl₂, 0.05% (v/v) Tritton X-100, 400 µM ATP, 32% (v/v) PEG600, 50 pmol donor (P-anti-PEA2-thio5-P, see Table 1), 80 ng of acceptor DNA (single-stranded or double-stranded, N50, N100, M13FR; dsDNA will be mentioned later), and 3 µg of SDL was prepared and incubated at 37°C for 1 hour. 1 µL of the reaction solution was collected and mixed with 10 µL of formamide, and the resultant was incubated at 70°C for 5 minutes, and then loaded to 10% Novex TBE-Urea Gel. After electrophoresis, the gel was stained with SYBR Gold and photographed.
   Double-stranded DNA models were prepared as follows: 50 µL of a reaction solution containing 5 µL of ExTaq Buffer, 4 µL of 2.5 mM dNTPs, 20 pmol M13F and M13RV (see Table 1), 10 pg of pUC19 vector, and 5 U of ExTaq was prepared and incubated at 95°C for 1 minute, followed by 30 cycles of three-step incubation at 95°C for 15 seconds, at 55°C for 30 seconds, and at 72°C for 30 seconds, and final incubation at 72°C for 1 minute. 1 µL of Exonuclease I was added to the reaction solution, and the resultant was then incubated at 37°C for 15 minutes. Then, 1 µL of Klenow fragment (New England Biolabs Inc. (NEB)) was added, if necessary, to the reaction solution, and the resultant was further incubated for 15 minutes. Subsequently, DNA was purified using QiaQuick PCR purification kit (Qiagen N.V.).

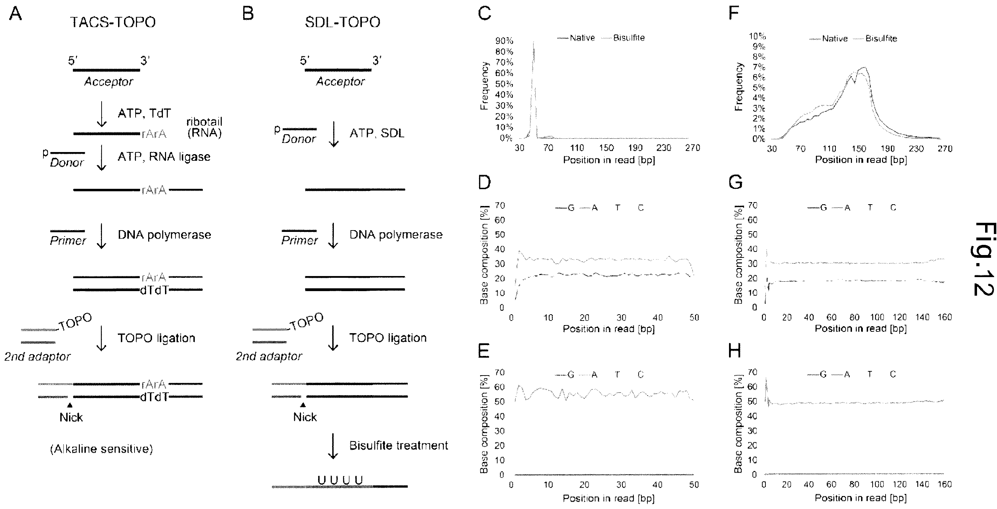

[Figure 12] Figure 12 is a diagram showing the preparation of a sequencing library from ssDNA (SDL-TOPO scheme).
   A and B. TACS-TOPO (A) and SDL-TOPO (B) library preparation schemes. Bisulfite treatment cannot be applied to the TACS-TOPO library because this library contains RNA.
   C to H. Results of analyzing sequence reads of libraries prepared by the SDL-TOPO method. Results of sequencing libraries prepared from N50 (C to E) and blood-derived cell-free DNA (G and H) with (D and E) or without (G and H) bisulfite treatment. As is evident, the bisulfite treatment resulted in large change in base composition. The sequence of a complementary strand of the bisulfite-treated DNA was analyzed. Specifically, the bisulfite treatment drastically decreased the content of the base G and instead increased the content of the base A. The details of the library preparation (SDL-TOPO method) are as follows.

### <SDL-TOPO method>

25 µL of a reaction solution containing target single-stranded DNA, 2.5 µL of 10 × SDL buffer, 1 µL of 100 mM ATP, 25 pmol adapter (P-AR-thio5-NH2 for native and 25 µM P-AR-Bs-thio5-NH2 in the case of performing bisulfite treatment, see Table 1), 7.5 µL of PEG600, and 3 µg of SDL was provided and incubated at 37°C for 1 hour. After the enzyme was inactivated by heating at 95°C for 5 minutes, 5 µL of 10 × ExTaq buffer, 4 µL of 2.5 mM dNTP, 50 pmol primer oligo (NH2-AF for native and NH2-AF-Bs in the case of performing bisulfite treatment, see Table 1), and 1 µL of Hot-Start Gene Taq (Nippon Gene Co., Ltd., Toyama, Japan) were added to the reaction solution to provide 50 µL of a reaction solution. This reaction solution was sequentially incubated at 95°C for 3 minutes, at 45°C for 5 minutes, and at 65°C for 15 minutes. Next, 45 µL of Buffer B2 (Qiagen N.V.) and 5 µL of Proteinase K (Qiagen N.V.) were added to the reaction solution, and the resultant was incubated at 50°C for 15 minutes. 100 µL of AxyPrep PCR clean was added to the reaction solution, and the resultant was incubated at room temperature for 5 minutes. Then, the beads were washed twice with 200 µL of a 50-bp cutoff solution. After the beads were rinsed with 70% (v/v) ethanol, DNA was eluted with 45 µL of 10 mM Tris-Acetate.

To 45 µL of the solution containing the purified DNA, 5 µL of 10 × ExTaq buffer and 1 µL of 150 ng/µL VOC were added, and the resultant was incubated at 25°C for 15 minutes. Here, VOC for bisulfite was used when bisulfite treatment was performed later; and VOC for native was used in the other cases. After this adapter ligation reaction, 45 µL of Buffer B2 (Qiagen N.V.) and 5 µL of Proteinase K (Qiagen N.V.) were added to the reaction solution, and the resultant was incubated at 50°C for 15 minutes. Next, 50 µL of AxyPrep PCR clean was added to the reaction solution, and the resultant was incubated at room temperature for 5 minutes. The beads were washed twice with 200 µL of a 50-bp cutoff solution and once with 200 µL of 70% (v/v) ethanol, followed by the elution of a library with 20 µL of 10 mM Tris-Acetate. The eluted library was subjected, if necessary, to bisulfite treatment.

The obtained library was supplemented with 25 µL of 2 × KOD One (Toyobo Co., Ltd.) and 10 pmol each of Primer-3 and Index-1 (Table 1). Then, the reaction product was incubated at 95°C for 1 minute, followed by an appropriate number of cycles of three-step incubation at 95°C for 15 seconds, at 55°C for 15 seconds, and 72°C for 30 seconds, and final incubation at 72°C for 3 minutes. The amplified library was loaded to 2% E-Gel Ex to confirm an amplification status.

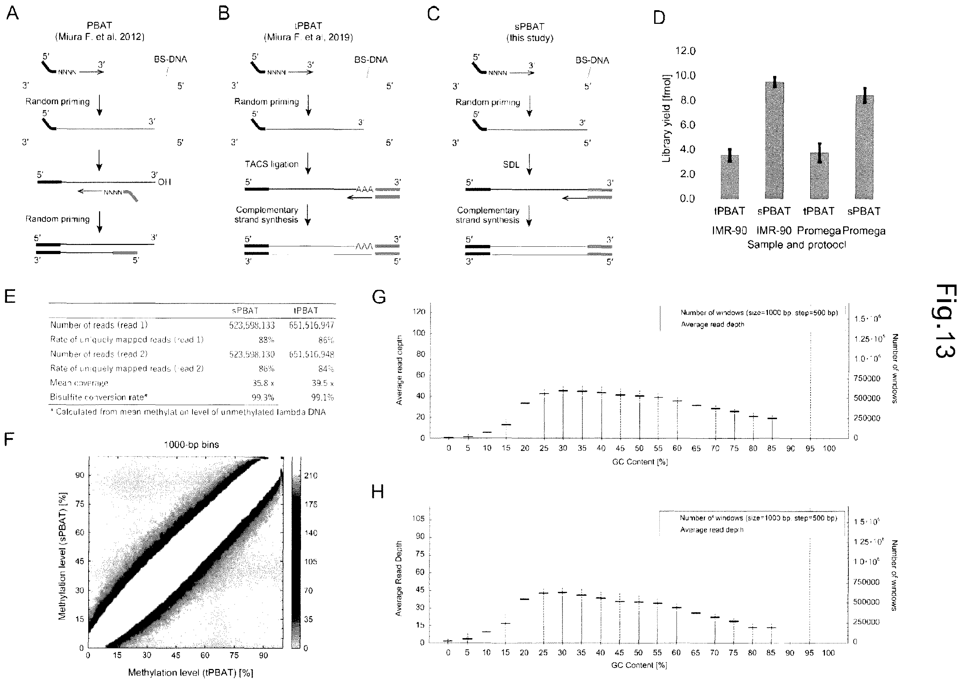

[Figure 13] Figure 13 is a diagram showing library preparation for whole-genome bisulfite sequencing using SDL.

A to C. Summary of library preparation in a post-bisulfite adapter tagging (PBAT) strategy. In the original version of the PBAT method (PBAT or rPBAT method; see Miura F. et al., 2012, (Non Patent Literature 14 listed above)), two random priming (RP) reactions were performed for introducing adapter sequences (A). However, repeating RP leads to shorter inserts. Hence, the second RP was replaced with TACS ligation (tPBAT; see Miura F. et al., 2019 (Non Patent Literature 7 listed above)) in the second version of PBAT (B). The TACS ligation of tPBAT was further replaced with SDL to achieve a new protocol sPBAT (C).

D. Comparison of the library yields between sPBAT and tPBAT. Genomic DNA derived from IMR-90 cells and human female peripheral blood was used as models. Average yields from three experiments were indicated. The error bar depicts divergence in results (standard deviation). The details of the sPBAT protocol are as follows.

### <sPBAT protocol>

5 µL of 10 × NEBuffer 2, 4 µL of 2.5 mM dNTP, and 1 µl of 100 µM PEA2-N4 were added to the bisulfite-treated DNA (BS-DNA), and the resultant was incubated at 95°C for 3 minutes and at 4°C for 5 minutes. Random priming was started by the addition of 1 µL of 5 mg/ml Klenow fragment exo(-). The reaction solution was incubated at 4°C for 15 minutes, and after the reaction temperature was increased to 37°C at a rate of +1°C/min, the reaction solution was further incubated at 37°C for 30 minutes. The reaction was terminated by incubation at 70°C for 10 minutes. Subsequently, 50 µL of AxyPrep PCR clean was added to the reaction solution, and the resultant was incubated at room temperature for 5 minutes. The beads were washed twice with 200 µL of a 300-bp cutoff solution and once with 200 µL of 70% ethanol, followed by the elution of the purified DNA with 10 µL of 10 mM Tris-Acetate, pH 8.0.

2.5 µL of 10 × SDL, 1 µL of 100 mM ATP, 25 pmol adapter (P-PEA1-thio5-NH2), and 7.5 µL of PEG600 were added to 10 µL of the purified DNA, and the resultant was incubated at 95°C for 5 minutes. Subsequently, the reaction solution was equilibrated at 37°C for 5 minutes. Subsequently, 5 µg of SDL was added to the reaction solution, and the resultant was incubated at 37°C for 1 hour. The reaction was terminated by heating at 95°C for 5 minutes. Then, 25 µL of water and 50 µL of AxyPrep PCR clean were added to the reaction solution, and the resultant was incubated at room temperature for 5 minutes. The beads were washed with 70% ethanol, followed by the elution of a library with 25 µL of 10 mM Tris-Acetate, pH 8.0.

To this purified DNA, 25 µL of 2 × KOD One (Toyobo Co., Ltd.) and 10 pmol each of Primer-3 and Index-1 (Table 1) were added to prepare 50 µL of a reaction solution. Next, the reaction solution was incubated at 95°C for 1 minute, followed by two cycles of three-step incubation at 95°C for 15 seconds, at 55°C for 15 seconds, and at 72°C for 30 seconds, plus final incubation at 72°C for 1 minute. 50 µL of AxyPrep PCR clean was added to the reaction solution, and the resultant was incubated at room temperature for 5 minutes. Then, the beads were washed twice with 200 µL of a 400-bp cutoff solution and once with 200 µL of 70% ethanol, followed by the elution of the finally purified library with 20 µL of 10 mM Tris-Acetate, pH 8.0. The molar concentration of the library was determined using Library Quantitation Kit from Takara Bio Inc.

E. Basic statistics of methylome data of the IMR-90 libraries. Only the libraries prepared from IMR-90 were sequenced.

F. Plot in which DNA methylation levels calculated in the IMR-90 libraries were compared in the whole genome. The mean methylation levels of CpG sites in 1000-bp bins were compared between sPBAT and tPBAT. Only the CpG sites covered with at least five reads were used in this calculation.

G and H. GC content-dependent coverage bias in the IMR-90 libraries was compared. The libraries obtained by tPBAT (G) and sPBAT (H) were indicated. The abscissa of the graph depicts the GC contents of the 1000-bp bins, and the ordinate of the graph depicts the frequencies of coverage (box plot in the orange boxes; left scale) and the genome distributions of the bins (green bars; right scale).

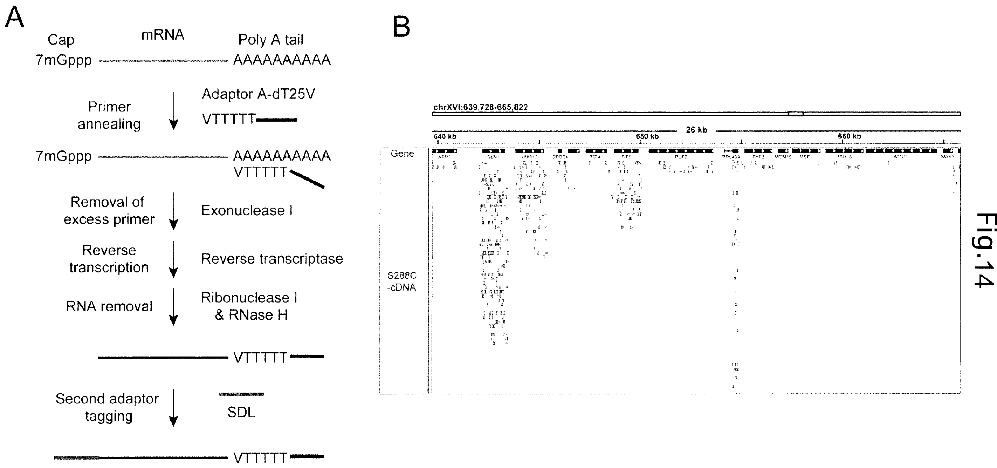

[Figure 14] Figure 14 is a diagram showing that SDL is effective for the analysis of cDNA.
A. Procedures of preparing a library of cDNA tailed at both ends with adapter sequences for amplification from total RNA.
B. Browser shots depict the distribution of reads mapped on reference genome. Integrative Genomics Viewer (IGV) was used. The details of the protocol of library preparation are as follows.

### <Protocol of library preparation: Long-read sequencing of budding yeast cDNA>

### (i) Total RNA extraction

Total RNA was extracted by a previously reported method (see Miura F. et al., 2008 (Non Patent Literature 15 listed above)). The extraction was performed from a budding yeast strain S288C in the growth phase cultured in YPD medium.

### (ii) cDNA synthesis

cDNA was synthesized by reverse transcription reaction with SuperScript IV (Thermo Fisher Scientific Inc.) combined with enzymatic removal of redundant primers with exonuclease I. 50 pmol PEA1-dT20V (Table 1) was added to 3 µg of the total RNA, which was then adjusted to 10 µL with water, and the resultant was heated at 70°C for 5 minutes. After subsequent cooling on ice for 5 minutes, 4 µL of 5 × SSIV buffer, 4 µL of 2.5 mM dNTPs, 1 µL of 100 mM DTT, and 1 µL of exonuclease I (NEB) were added to the reaction solution, and the resultant was incubated at 37°C for 15 minutes. This reaction solution was supplemented with 1 µL of 200 U/µL SuperScript IV, and the resultant was incubated at 42°C for 15 minutes, at 50°C for 45 minutes, and at 80°C for 10 minutes. To this reaction solution, 1 µL of 50 U/µL RNase If (NEB) was added and incubated at 37°C for 30 minutes and at 80°C for 10 minutes. Furthermore, 30 µL of water and 50 µL of AxyPrep PCR clean were added to the reaction solution, and the resultant was incubated at room temperature for 5 minutes. Then, the beads were washed twice with 200 µL of a 300-bp cutoff solution and once with 200 µL of 70% (v/v) ethanol, followed by the elution of the purified cDNA with 10 µL of 10 mM Tris-Acetate, pH 8.0.

### (iii) Second adapter ligation with SDL

25 µL of a reaction solution containing 10 µL of the purified cDNA solution as well as 2.5 µL of 10 × SDL buffer, 1 µL of 100 mM ATP, 25 pmol P-anti-PEA2-thio5-P, 10 µL of PEG600, and 3 µg of SDL was provided. The reaction solution was then incubated at 37°C for 1 hour at 80°C for 10 minutes. Next, 5 µL of 10 × ExTaq buffer, 4 µL of 2.5 mM dNTP, 50 pmol PEA2, and 1 µL of 5 U/µL ExTaq HS were added to the reaction solution, and the resultant was incubated at 95°C for 3 minutes, at 55°C for 5 minutes, and at 72°C for 30 minutes. 45 µL of Buffer B2 and 5 µL of proteinase K were added to the reaction solution, and the resultant was incubated at 50°C for 15 minutes. Then, 50 µL of AxyPrep PCR clean was added to the reaction solution, and the resultant was incubated at room temperature for 5 minutes. Furthermore, the beads were washed twice with 200 µL of a 300-bp cutoff solution and once with 200 µL of 70% (v/v) ethanol. Finally, a library was eluted with 25 µL of 10 mM Tris-Acetate, pH 8.0.

### (iv) PCR amplification of cDNA

To the adapter-ligated cDNA eluted in 25 µL, 25 µL of 2 × KOD One and 10 pmol each of Primer-3 and Index-1 were added. The reaction solution was incubated at 95°C for 1 minute, followed by 12 cycles of three-step incubation at 95°C for 15 seconds, at 55°C for 30 seconds, and at 72°C for 3 minutes. After final incubation at 72°C for 3 minutes, the reaction solution was mixed with 50 µL of AxyPrep PCR clean, and the resultant was incubated at room temperature for 5 minutes. The beads were washed twice with 200 µL of a 300-bp cutoff solution and once with 200 µL of 70% (v/v) ethanol, followed by the elution of the amplified cDNA with 20 µL of 10 mM Tris-Acetate, pH 8.0. The concentration of the cDNA thus amplified was determined with Qubit dsDNA BR Kit.

### (v) Nanopore sequencing

Sequencing was performed using 800 ng of the amplified cDNA. The sequencing was carried out in accordance with the protocol of Ligation sequencing amplicons using Flongle Flow Cell (FLO-FLG001, Oxford Nanopore Technologies plc) combined with Ligation Sequencing Kit (SQK-LSK110, Oxford Nanopore Technologies plc). After reads output in the fastq format were mapped onto budding yeast reference genome sacCer3 using CLC Genomic Workbench (Qiagen N.V.), the alignment was exported in the BED format and visualized by IGV.

### Description of Embodiments

Hereinafter, the present invention will be described in detail. The scope of the present invention is not bound by the description, and embodiments given below and other embodiments can be carried out through appropriate changes or modifications without departing from the spirit of the present invention. The present application claims priority to Japanese Patent Application No. 2023-088967 (filed on May 30, 2023), the content of which is incorporated herein in its entirety.

All publications, for example, prior art documents, laid-open publications, patents, and other patent documents, cited herein are incorporated herein by reference.

### 1. Summary of present invention

An enzyme with strong single-stranded DNA ligation activity would be useful for developing various molecular biology applications. However, previously known enzymes exhibit only limited activity. The present inventor has identified an enzyme with excellent single-stranded DNA ligation activity by searching databases for an amino acid sequence group homologous to "TS2126 RNA ligase" (see Non Patent Literature 9 listed above), which has the highest single-stranded DNA ligation activity among the known enzymes. In particular, ligase derived from Thermus phage phiLo (Tph ssDNA ligase, GenBank Accession No: AYJ73970) exhibited evidently higher single-stranded DNA ligation activity than that of the TS2126 RNA ligase. Enzyme proteins with single-stranded DNA ligation activity, including this Tph ssDNA ligase, found by the present inventor are useful for developing various applications including library preparation for sequencing. In the entire present application including the present invention and the present specification, the enzyme (Tph ssDNA ligase, GenBank Accession No: AYJ73970) is referred to as "SDL".

### 2. Composition for ligating single-stranded DNA, etc.

The composition for ligating single-stranded DNA according to the present invention is a composition which is used for ligating at least one single-stranded DNA as an acceptor to at least one single-stranded DNA as a donor, the composition comprising the following protein (a) and/or (b) (protein having a function as an enzyme with single-stranded DNA ligation activity; hereinafter, also referred to as the "protein according to the present invention").
(a) A protein comprising an amino acid sequence represented by SEQ ID NO: 2 or 4.
(b) A protein which comprises an amino acid sequence having 70% or higher identity to the amino acid sequence represented by SEQ ID NO: 2 or 4 and has single-stranded DNA ligation activity.

The amino acid sequence represented by SEQ ID NO: 2 is registered under GenBank (https://www.ncbi.nlm.nih.gov/genbank/) Accession No: AYJ37970. Examples of the nucleotide sequence encoding the amino acid sequence represented by SEQ ID NO: 2 include various nucleotide sequences synthesized on the basis of this amino acid sequence and preferably include the nucleotide sequence represented by SEQ ID NO: 1.

The amino acid sequence represented by SEQ ID NO: 4 is registered under GenBank Accession No: WP_119360902. Examples of the nucleotide sequence encoding the amino acid sequence represented by SEQ ID NO: 4 include various nucleotide sequences synthesized on the basis of this amino acid sequence and preferably include the nucleotide sequence represented by SEQ ID NO: 3.

The protein having the amino acid sequence represented by SEQ ID NO: 2 or 4 has been identified as a protein having a function as an enzyme with single-stranded DNA ligation activity for the first time by the present inventor.

The protein according to the present invention is not limited to a protein consisting of the amino acid sequence represented by SEQ ID NO: 2 or 4 and also encompasses a protein comprising the amino acid sequence represented by SEQ ID NO: 2 or 4.

Preferred examples of the protein according to the present invention also include proteins which comprise an amino acid sequence derived from the amino acid sequence represented by SEQ ID NO: 2 or 4 by the deletion, substitution, or addition of one or several amino acids and have single-stranded DNA ligation activity (so-called mutant proteins).

In this context, the "amino acid sequence derived by the deletion, substitution, or addition of one or several amino acids" is preferably an amino acid sequence with, for example, approximately 1 to 10, preferably 1 to several, 1 to 5, 1 to 4, 1 to 3, 1 or 2, or 1 amino acid(s) deleted, substituted, or added. A mutation such as this deletion, substitution, or addition can be introduced using a kit for mutation introduction that exploits a site-directed mutagenesis method, for example, GeneTailor(TM) Site-Directed Mutagenesis System (Invitrogen Corp.) or TaKaRa Site-Directed Mutagenesis System (e.g., Prime STAR(R) Mutagenesis Basal kit or Mutan(R)-Super Express Km; manufactured by Taka Bio Inc.). Successful introduction of the mutation, i.e., deletion, substitution, or addition, can be confirmed by use of various amino acid sequencing methods and structural analysis methods based on X ray and NMR, for example.

Preferred examples of the protein according to the present invention also include proteins which comprise an amino acid sequence having 70% or higher identity (homology) to the amino acid sequence represented by SEQ ID NO: 2 or 4 and have single-stranded DNA ligation activity (so-called mutant protein).

In this context, the identity is more preferably 75% or higher, 80% or higher, 81% or higher, 82% or higher, 83% or higher, 84% or higher, 85% or higher, 86% or higher, 87% or higher, 88% or higher, 89% or higher, 90% or higher, 91% or higher, 92% or higher, 93% or higher, 94% or higher, 95% or higher, 96% or higher, 97% or higher, 98% or higher, or 99% or higher.

In the present invention, the phrase "with single-stranded DNA ligation activity" or "having single-stranded DNA ligation activity" means having at least activity of directly ligating donor DNA and acceptor DNA, both of which are single-stranded DNA, to each other (ligation activity). In this context, the activity of directly ligating donor DNA and acceptor DNA to each other is meant to exclude activity of ligating single-stranded DNA, for example, donor DNA and acceptor DNA each ribotailed (ribotail: some RNA constituent bases such as adenylic acids) at one end (end to be attached to the partner). However, the protein according to the present invention may also have such activity without limitations. The protein according to the present invention can be confirmed to "have single-stranded DNA ligation activity" by the sPBAT method, which will be mentioned later in Example.

The protein according to the present invention may be a peptide derived from a natural product or may be artificially obtained by chemical synthesis, without limitations.

The protein derived from a natural product may be obtained directly from the natural product by a recovery method and a purification method known in the art, or may be prepared in a genetic engineering manner using a gene encoding the amino acid sequence of the protein (also including the mutant proteins mentioned above) by a gene recombination technique known in the art. The protein may be obtained, for example, by inserting the encoding gene to various expression vectors or the like, transfecting cells with the vectors, and allowing the cells to express the protein, which is obtained by a recovery method and a purification method known in the art. Alternatively, the protein according to the present invention may be produced by a cell-free protein synthesis system using a commercially available kit, for example, a reagent kit PROTEIOS(TM) (Toyobo Co., Ltd.), TNT(TM) System (Promega Corp), a synthesizer PG-Mate(TM) (Toyobo Co., Ltd.), or RTS (Roche Diagnostics), and obtained by a recovery method and a purification method known in the art, without limitations.

The chemically synthesized protein can be obtained by use of a protein synthesis method known in the art. Examples of the synthesis method include azide methods, acid chloride methods, acid anhydride methods, mixed acid anhydride methods, DCC methods, active ester methods, carboimidazole methods, and oxidation-reduction methods. Any of solid-phase synthesis methods and liquid-phase synthesis methods can be applied to the synthesis. A commercially available protein synthesizer may be used. After synthesis reaction, the protein can be purified by a combination of purification methods known in the art, such as chromatography.

In the present invention, a derivative of the protein according to the present invention mentioned above can be included together with or instead of the protein. The derivative is meant to include every product that may be prepared from the protein. Examples thereof include products obtained by partial substitution of constituent amino acids with non-natural amino acids, and products obtained by partial chemical modification of constituent amino acids (mainly, their side chains).

In the present invention, a salt of the protein according to the present invention mentioned above and/or the derivative of the protein can be included together with or instead of the protein and/or the derivative. The salt is preferably a physiologically acceptable acid-addition salt or basic salt. Examples of the acid-addition salt include salts with inorganic acids such as hydrochloric acid, phosphoric acid, hydrobromic acid, and sulfuric acid, and salts with organic acids such as acetic acid, formic acid, propionic acid, fumaric acid, maleic acid, succinic acid, tartaric acid, citric acid, malic acid, oxalic acid, benzoic acid, methanesulfonic acid, and benzenesulfonic acid. Examples of the basic salt include salts with inorganic bases such as sodium hydroxide, potassium hydroxide, ammonium hydroxide, and magnesium hydroxide, and salts with organic bases such as caffein, piperidine, trimethylamine, and pyridine.

The salt can be prepared using an appropriate acid such as hydrochloric acid, or an appropriate base such as sodium hydroxide. The salt can be prepared, for example, by treatment using a standard protocol in water or in a liquid containing an inert water-miscible organic solvent such as methanol, ethanol, or dioxane.

The single-stranded DNA as an acceptor and the single-stranded DNA as a donor to be ligated by the composition for ligating single-stranded DNA according to the present invention are, for example, acceptor DNA of preferably 12 or more bases (which may be 14 or more bases, 16 or more bases, 18 or more bases, 20 or more bases, 22 or more bases, 24 or more bases, 26 or more bases, 28 or more bases, 30 or more bases, 32 or more bases, 34 or more bases, 36 or more bases, 38 or more bases, or 40 or more bases) long, and donor DNA of preferably 10 or more bases (which may be 11 or more bases, 12 or more bases, 13 or more bases, 14 or more bases, 15 or more bases, 16 or more bases, 17 or more bases, 18 or more bases, 19 or more bases, or 20 or more bases) long. When the base lengths of the acceptor DNA and the donor DNA fall within the above ranges, stable single-stranded DNA ligation activity can be achieved.

The composition for ligating single-stranded DNA according to the present invention preferably further comprises PEG without limitations. PEG is not limited by its type, and one or more members selected from, for example, PEG400, PEG600, PEG1540, PEG2000, PEG4000, and PEG6000 can be used. PEG400 and PEG600 are preferably used at, for example, 30 to 60% by weight (which may be 30 to 50% by weight or 30 to 40% by weight) with respect to the composition; PEG1540 and PEG2000 are preferably used at, for example, 20% by weight or more (which may be 25% by weight or more or 30% by weight or more) with respect to the composition; and PEG4000 and PEG6000 are preferably used at, for example, 15% by weight or more (which may be 20% by weight or more or 25% by weight or more) with respect to the composition.

The reaction temperature in the ligation reaction of the single-stranded DNA as an acceptor and the single-stranded DNA as a donor using the composition for ligating single-stranded DNA according to the present invention is not limited and is, for example, preferably 20 to 49°C (which may be 30 to 45°C, 35 to 45°C, or 37 to 42°C), more preferably 37°C.

The pH of the reaction solution in the ligation reaction is not limited and is, for example, preferably pH 7.5 to 9.5 (which may be pH 8.0 to 9.5 or pH 7.5 to 9.0).

Examples of other components that may be further contained in the reaction solution include, but are not limited to, magnesium chloride (MgCl₂), ammonium sulfate, sodium chloride, potassium chloride, and ATP. MgCl₂ is desirably an essential component in the reaction solution and preferably has a concentration of, for example, 1.0 to 20.0 mM (which may be 2.5 to 20 mM or 5 to 20 mM). Ammonium sulfate preferably has a concentration of, for example, less than 20 mM (which may be less than 16 mM or less than 12 mM), and sodium chloride and potassium chloride preferably have a total concentration of 50 mM or lower. These other components may be contained in the reaction solution in the ligation reaction or may be contained in advance as constituents in the composition for ligating single-stranded DNA according to the present invention, without limitations.

The present invention can also provide a method for producing ligated single-stranded DNA or a method for ligating single-stranded DNA, comprising bringing the protein according to the present invention mentioned above (and furthermore, the composition for ligating single-stranded DNA according to the present invention) into contact with at least one single-stranded DNA as an acceptor and at least one single-stranded DNA as a donor, to ligate the acceptor DNA to the donor DNA. The detailed description about the composition for ligating single-stranded DNA according to the present invention mentioned above can be appropriately applied to various conditions for carrying out these methods.

The present invention encompasses, as in the aspects directed to the composition for ligating single-stranded DNA mentioned above, aspects directed to a single-stranded DNA ligation agent and a kir for ligating single-stranded DNA which are used for ligating at least one single-stranded DNA as an acceptor to at least one single-stranded DNA as a donor. Various descriptions in the composition for ligating single-stranded DNA mentioned above can be appropriately applied to the details of contained components and constituents of the ligation agent or the kit.

The present invention also encompasses aspects directed to the protein according to the present invention for use in the method for producing ligated single-stranded DNA or the method for ligating single-stranded DNA mentioned above, use of the protein according to the present invention in the method, and the like.

The present invention further encompasses aspects directed to a method for preparing a double-stranded DNA library from a single-stranded DNA fragment using the protein according to the present invention, and aspects directed to a double-stranded DNA library obtainable by the preparation method.

One aspect of the method for preparing a double-stranded DNA library (preparation method I) is, for example, a method for preparing a double-stranded DNA library from a single-stranded DNA fragment, the preparation method comprising the steps of:
(A) ligating a 5'-terminally phosphorylated single-stranded adapter sequence to the target single-stranded DNA fragment;
(B) performing DNA replication beginning at the adapter of the ligated sequence to form double-stranded DNA;
(C) ligating a topoisomerase-complexed double-stranded adapter sequence to ends tailed with no adapter sequence of the double-stranded DNA; and
(D) using the ligated double-stranded DNA as a template in PCR to obtain a library comprising a plurality of the double-stranded DNA, wherein
the ligation in the step (A) is performed by a ligation method using
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

More specifically, one aspect of the method for preparing a double-stranded DNA library (preparation method II) is, for example, a method for preparing a double-stranded DNA library from a single-stranded DNA fragment, the preparation method comprising the steps of:
(i) ligating a 5'-terminally phosphorylated single-stranded adapter sequence A1 to the 3' end of a single-stranded DNA fragment dephosphorylated at both ends to form adapter-tailed single-stranded DNA;
(ii) annealing a single-stranded adapter sequence A2 to the adapter sequence A1 moiety contained in the adapter-tailed single-stranded DNA, followed by extension from the 3' end of the adapter sequence A2 with DNA polymerase to form adapter-tailed double-stranded DNA;
(iii) reacting a double-stranded adapter sequence B1 with topoisomerase to form a topoisomerase-complexed double-stranded adapter sequence B2, wherein the double-stranded adapter sequence B1 is phosphorylated at only one 5' end and has a topoisomerase recognition sequence in a region containing a 3' end complementary to this phosphorylated 5' end, and wherein the topoisomerase-complexed double-stranded adapter sequence B2 has the topoisomerase conjugated with the 3' end of the topoisomerase recognition sequence;
(iv) ligating the topoisomerase-complexed double-stranded adapter sequence B2 to ends tailed with no adapter sequence of the adapter-tailed double-stranded DNA to form both-terminally adapter-tailed double-stranded DNA; and
(v) using the both-terminally adapter-tailed double-stranded DNA as a template in PCR to obtain a library comprising a plurality of the double-stranded DNA, wherein
the ligation and the formation in the step (i) are performed by a ligation method using
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

In this context, the description about the protein according to the present invention mentioned above as well as the description about the method for producing ligated single-stranded DNA and the description about the method for ligating single-stranded DNA mentioned above can be appropriately applied to the details of the protein (a) and/or (b) and the ligation method using the protein in each aspect of the method for preparing a double-stranded DNA library (preparation method I or II) described above. Each aspect of the method (preparation method I or II) described above is not limited by comprising each of the steps mentioned above and may comprise other optional steps.

Hereinafter, embodiments of the preparation method II in each aspect of the method for preparing a double-stranded DNA library (preparation method I or II) described above will be described in more detail. The details of embodiments of the preparation method I can be understood with appropriate reference to the details of the embodiments of the preparation method II.

### Step (i)

The "single-stranded DNA fragment" serving as so-called insert DNA for use in library preparation is dephosphorylated at both ends (5' end and 3' end). Next, the "single-stranded adapter sequence A1" which is 5'-terminally phosphorylated single-stranded DNA is ligated to the 3' end of the single-stranded DNA fragment. The ligation is performed by the ligation method using the protein according to the present invention, as mentioned above. The "adapter-tailed single-stranded DNA" is formed through the ligation. The details (including various reaction conditions and the like) of the formation process of the adapter-tailed single-stranded DNA in the step (i) can be appropriately selected and are not particularly limited.

### Step (ii)

This step is of converting the adapter-tailed single-stranded DNA formed in the step (i) to double-stranded DNA. Specifically, the "single-stranded adapter sequence A2" which is single-stranded DNA is annealed to the adapter sequence A1 moiety in the formed adapter-tailed single-stranded DNA. The single-stranded adapter sequence A2 (particularly, its 3'-terminal moiety) preferably has a nucleotide sequence complementary at least partially to the nucleotide sequence of the adapter sequence A1 moiety. After the annealing, extension from the 3' end of the adapter sequence A2 is caused by DNA polymerase. The DNA polymerase is not limited and is preferably Taq DNA polymerase or the like. In this way, double-stranded DNA, i.e., the "adapter-tailed double-stranded DNA", is formed. For the details (including various reaction conditions and the like) of the formation process of the adapter-tailed double-stranded DNA in the step (ii), some steps of the "TACS-T4" library preparation method disclosed in Non Patent Literature 1 listed above (BMC Biology, 2021) can be appropriately referred to.

### Step (iii)

This step is of preparing the "double-stranded adapter sequence B1" serving as an adapter sequence of double-stranded DNA for use in the so-called second ligation (specifically, the next step (iv)). First, one single-stranded DNA in the double-stranded adapter sequence B1 is phosphorylated at its 5' end, while the other single-stranded DNA is not phosphorylated at its 5' end. Furthermore, a region containing a 3' end complementary to the phosphorylated 5' end has a topoisomerase recognition sequence. The topoisomerase is preferably topoisomerase I, more preferably topoisomerase I derived from vaccinia virus (VTopoI). Examples of the VTopoI recognition sequence include, but are not limited to, 5'-CCTTT-3' and 5'-(C/T)CCTT-3'. VTopoI specifically recognizes the sequence of these five bases and forms a covalent bond with the phosphate group of 3'-T. A nucleotide sequence located on the 3' side from 3'-T in the recognition sequence is cleaved along with the formation. A region containing the phosphorylated 5' end of the double-stranded adapter sequence B1 preferably has a nick (cut in the nucleotide sequence), and this nick preferably resides at, for example, a base on the 5' side of the sequence complementary to the VTopoI recognition sequence, more preferably at a base on the 5' side by one base of the complementary sequence, without limitations. In the presence of the nick, the nucleotide sequence located on the 3' side from 3'-T in the recognition sequence is cleaved, together with a sequence comprising a sequence complementary to the cleaved sequence, from the double-stranded adapter sequence B1. In this way, the "topoisomerase-complexed double-stranded adapter sequence B2" is formed. When the double-stranded adapter sequence B1 has the nick, the nick or a deleted portion corresponding to the nick preferably remains in the double-stranded adapter sequence B2.

### Step (iv)

The topoisomerase-complexed double-stranded adapter sequence B2 formed in the step (iii) is ligated to ends tailed with no adapter sequence, of the adapter-tailed double-stranded DNA formed in the step (ii). The topoisomerase (VTopoI) has activity of ligating the phosphate group of 3'-T (i.e., the phosphorylated 3' end) covalently bound with the topoisomerase to the dephosphorylated 5' end (i.e., the hydroxy group of the 5' end) of the adapter-tailed double-stranded DNA. This forms the "both-terminally adapter-tailed double-stranded DNA". On the other hand, the topoisomerase-complexed double-stranded adapter sequence B2 is not ligated in principle to an unreacted double-stranded adapter (both strands have a phosphorylated 5' end) formed by the annealing of the single-stranded adapter sequence A1 and the single-stranded adapter sequence A2 mentioned above (i.e., the annealing of free sequences A1 and A2). Hence, the dimerization of the unreacted adapter and the adapter sequence B2 is effectively suppressed. The dimer might be formed by accident. In such a case, the dimer can be degraded by the addition of DNA endonuclease to the reaction system after formation of the "both-terminally adapter-tailed double-stranded DNA" to create a state similar to that in which no dimer is formed, for example, when the double-stranded adapter sequence B1 has a nick (the nick or a deleted portion corresponding to the nick also remains in the double-stranded adapter sequence B2), as mentioned in the step (iii), and the single-stranded adapter sequence A1 has uracil in the sequence. Examples of the DNA endonuclease include enzymes having activity of hydrolyzing a N-glycoside bond in uracil or activity of making a nick in the DNA backbone on the 5' side of uracil. Such DNA endonuclease is preferably, for example, UDG (uracil DNA glycosylate), APE 1 (human-derived apurinic/apyrimidinic (AP) endonuclease), or EndoQ (endonuclease Q). The uracil removal by the DNA endonuclease, or a nick near uracil and the nick present in the double-stranded adapter sequence B2 (more specifically, the nick present at the ligation site to the double-stranded adapter sequence B2) degrade (dissociate) the formed adapter dimer, which can consequently be removed from the reaction system. "dSpacer" may be introduced as spacer-modified oligo DNA instead of uracil in the single-stranded adapter sequence A1. In this context, the spacer-modified oligo DNA, an abasic structure, can be introduced into the backbone with phosphodiester bonds of a DNA strand and includes a type deficient only in base and a type deficient both in base and in deoxyribose ring. The number of its spacer arms can be variously selected. dSpacer is deficient in base moiety and not only can destabilize the double strands of a sequence moiety with the insert but can control the stability of the double strands depending on a site or a structure with the insert. As described above, the introduction of dSpacer is effective for preventing the formation of an adapter dimer and can also facilitate removing the adapter dimer by enzymatic degradation. "Ethynyl dSpacer" (which has an alkyne structure attached to the 1'-position of a sugar where a base is attached in the normal oligo DNA strand) may be adopted instead of or in combination with the dSpacer.

### Step (v)

This step is of using the formed both-terminally adapter-tailed double-stranded DNA as a template in PCR to obtain a library comprising a plurality of the double-stranded DNA. PCR reaction conditions, purification conditions for the obtained PCR products (library), and the like can be appropriately set, and some steps of the "TACS-T4" library preparation method disclosed in Non Patent Literature 1 listed above (BMC Biology, 2021) can be appropriately referred to.

Hereinafter, the present invention will be more specifically described with reference to Example. However, the present invention is not limited by the example.

### [Example 1]

### <Description of material and method>

### (1) Artificial gene synthesis, protein expression, and purification thereof

A gene encoding each of proteins registered under GenBank (https://www.ncbi.nlm.nih.gov/genbank/) Accession Nos: AYJ37970, WP_119360902, RTT02525, WP_081892735, WP_110532308, and WP_126206484 was synthesized by Twist Biosciences (South San Francisco, CA) with optimization for the codon usage of an *E. coli* K-12 strain (SEQ ID NO: 1 (AYJ37970), SEQ ID NO: 3 (WP_119360902), SEQ ID NO: 5 (RTT02525), SEQ ID NO: 6 (WP_081892735), SEQ ID NO: 7 (WP_110532308), and SEQ ID NO: 8 (WP_126206484)). These genes were PCR-amplified and subcloned into pET28a-StrepSL (SEQ ID NO: 9), and insert sequences were confirmed by Sanger sequencing and then used in the induction of expression.

The culture of *E. coli* transformants obtained with the expression vector, the induction of protein expression, and the purification of the recombinant proteins were performed in accordance with a method used in the previous research on the purification of the TS2126 RNA ligase (see Non Patent Literature 1 listed above). Specifically, highly purified proteins were obtained by use of the dual affinity strategy using two tags, His6 and Strep tags (see Non Patent Literature 1 listed above).

### (2) Screening for enzyme with single-stranded DNA ligation activity

25 µL of a reaction solution containing 2 pmol acceptor DNA (N100, see Table 1 below), 25 pmol donor DNA (P-anti-PEA2-thio5-P, see Table 1 below), 400 µM ATP, 50 mM HEPES-KOH, pH 7.5, 0.5% (v/v) Triton X-100, 20% (w/v) PEG6000, and 2 µg of RNA ligase was provided and sequentially incubated at 37°C, at 45°C, at 55°C, and at 65°C each for 30 minutes. Then, the enzyme was inactivated by heating at 95°C for 5 minutes. Subsequently, 25 µL of water, 45 µL of Buffer B2 (Qiagen N.V., Hilden, Germany), and 5 µL of proteinase K (Qiagen N.V.) were added to the reaction solution, and the resultant was further incubated at 50°C for 15 minutes. 100 µL of isopropanol and 2 µL of Sera-Mag Carboxylate Magnetic Beads (Cytiva, Marlborough, MA) were added to the reaction solution and mixed therewith, and the resultant was left standing for 5 minutes. Then, the magnetic beads were washed with 70% (v/v) ethanol, followed by the elution of the purified DNA with 10 µL of 10 mM Tris-Acetate, pH 8.0. This DNA was applied to 10% denaturing acrylamide gel, which was then stained with SYBR Gold and then photographed.

### [Table 1]

**Table 1: Chemically synthesized oligonucleotides used in present Example**

| Name | Purpose | Purification grade | SEQ ID NO | Sequence and modification |
|---|---|---|---|---|
| N100 | Acceptor | OPC | 10 | NNNNNNNNNNNNNNNNNNNNNNNNNNNNNNNNNNNNNNNNNNNNNNNNNNNNNNNNNNNN NNNNNNNNNNNNNNNNNNNNNNNNNNNNNNNNNNNNNNNNNNNNNNNNNNNNNNNNNNNN |
| N50 | Acceptor | OPC | 11 | NNNNNNNNNNNNNNNNNNNNNNNNNNNNNNNNNNNNNNNNNNNNNNNNNN |
| FAM-N40 | Acceptor | OPC | 12 | [FAM]NNNNNNNNNNNNNNNNNNNNNNNNNNNNNNNNNNNNNNNN |
| FAM-rN40 | Acceptor | HPLC | 13 | [FAM]nnnnnnnnnnnnnnnnnnnnnnnnnnnnnnnnnnnnnnnn |
| M13FR | Acceptor | OPC | 14 | GTAAAACGACGGCCAGTGAATTCGAGCTCGGTACCCGGGGATCCTCTAGAGTCGACCTG CAGGCATGCAAGCTTGGCGTAATCATGGTCATAGCTGTTTCCTG |
| P-anti-PEA2-thio5-P | Donor | OPC | 15 | [PHO]AGATCGGAAGAGCGTCGTGTAGGGAAA*G*A*G*T*G*T[PHO] |
| P-anti-PEA2-thio5-FAM | Donor | OPC | 16 | [PHO]AGATCGGAAGAGCGTCGTGTAGGGAAA*G*A*G*T*G*T[FAM] |
| P-rN20-P | Donor | HPLC | 17 | [PHO]nnnnnnnnnnnnnnnnnnnn[PHO] |
| M13F | PCR primer | OPC | 18 | GTAAAACGACGGCCAG |
| M13RV | PCR primer | OPC | 19 | CAGGAAACAGCTATGAC |
| PEA1-3C2T-Ext | VOC | HPLC | 20 | GTGACTGGAGTTCAGACGTGTGCTCTTCCGATCCCCTTATTCCGATAGTG |
| PEA1-lT-3C2T-Ext-met | VOC | HPLC | 21 | GTGAMTGGAGTTMAGAMGTGTGMTMTTMMGATMTCCCTTATTCCGATAGTG |
| 1A3GlA-anti-PEA1 | VOC | HPLC | 22 | AGGGAGATCGGAAGAGCACACGTCTGAACTCCAGTCAC |
| Anti-Ext-1 | VOC | HPLC | 23 | CACTATCGGAA |
| P-AR-thio5-NH2 | SDL-TOPO | HPLC | 24 | [PHO]AGATCGGAAGAGCG*T*C*G*T*G[Am] |
| P-AR-Bs-thio5-NH2 | SDL-TOPO | HPLC | 25 | [PHO]AGACMGGAAGAGMG*C*M*G*C*G[Am] |
| NH2-AF | SDL-TOPO | HPLC | 26 | [Am]CACGACGCTCTTCCGATCT |
| NH2-AF-Bs | SDL-TOPO | HPLC | 27 | [Am]CGCGGCGCTCTTCCGGTCT |
| Primer-3 | SDL-TOPO | OPC | 28 | AATGATACGGCGACCACCGAGATCTACACTCTTTCCCTACACGACGCTCTTCCGATCT |
| Index-1 | SDL-TOPO | OPC | 29 | CAAGCAGAAGACGGCATACGAGATCGTGATGTGACTGGAGTTCAGACGTGTGCTCTTCC GATC |
| PEA2 | sPBAT | OPC | 30 | ACACTCTTTCCCTACACGACGCTCTTCCGATCT |
| PEA2-N4 | sPBAT | OPC | 31 | ACACTCTTTCCCTACACGACGCTCTTCCGATCTNNNN |

| | | | | |
|---|---|---|---|---|
| [Remarks] N: DNA of degenerate sequence of four types of bases n: RNA of degenerate sequence of four types of bases [FAM]: FAM fluorescent label [PHO]: Phosphorylation label *: Phosphorothioate bond [Am]: Amination modification M: 5-methyl cytosine (5mC) | | | | |

### (3) Preparation of sequencing library from single-stranded DNA

The details of sequencing library preparation are as follows.

Human serum was obtained from Kohjin Bio Co., Ltd (Saitama, Japan). Cell-free DNA (cfDNA) was purified from 5 µL of the serum by use of the previously described PPAP method (see Non Patent Literature 1 listed above). 25 µL of a reaction solution containing the purified cfDNA, 50 mM Tris-HCl, pH 8.5, 10 mM MgCl₂, 0.05% (v/v) Triton X-100, 400 µM ATP, 25 pmol adapter (P-anti-PEA2-thio5-P, see Table 1 above), 40% (v/v) PEG600, and 3 µg of the enzyme protein (screened for as an enzyme with single-stranded DNA ligation activity) was prepared and incubated at 37°C for 1 hour. After the enzyme was sterilized by heating at 95°C for 5 minutes, the reaction solution was complemented with 5 µL of 10 × ExTaq buffer, 4 µL of 2.5 mM dNTP, 50 pmol primer oligo (PEA2, see Table 1 above), and 1 µL of Hot-Start GeneTaq (Nippon Gene Co., Ltd., Toyama, Japan). The reaction was performed by sequential incubation at 95°C for 3 minutes, at 45°C for 5 minutes, and at 65°C for 15 minutes. The reaction was complemented with 5 µL of 500 mM EDTA-NaOH, pH 8.0 and 1 µL of 1 mg/ml VTopoI-oligonucleotide complex (VOC) prepared as previously described, followed by incubation at 25°C for 15 minutes.

Then, 50 µL of AxyPrep PCR Clean (Corning Inc., NY) was added to the reaction solution, and the resultant was incubated at ordinary temperature for 5 minutes. The beads were washed twice with 200 µL of a solution containing 32% (v/v) PEG400, 1 M NaCl, and 50 mM Tris-HCl, pH 8.0. The beads were rinsed with 70% (v/v) ethanol, followed by the elution of a library with 25 µL of 10 mM Tris-Acetate. 5 µL of 10 × ExTaq buffer, 4 µL of 2.5 mM dNTPs, 10 pmol each of Primer-3 and Index-1 (see Table 1 above), and 1 µL of ExTaq HS were added to the purified library. Then, the reaction solution was incubated at 72°C for 5 minutes and at 95°C for 1 minute, followed by 12 cycles of three-step incubation at 95°C for 15 seconds, at 55°C for 15 seconds, and at 72°C for 30 seconds, and final incubation at 72°C for 1 minute. The amplified library was loaded to 2% E-Gel Ex, and gel images were taken.

The details of formation of the VTopol-oligonucleotide complex (VOC) are as follows.

### <Formation of vaccinia virus topoisomerase I (VTopoI)-oligonucleotide complex (VOC)>

VTopoI and T4 polynucleotide kinase (T4 PNK) were purified by a method described in the previous report (see Non Patent Literature 16: Miura F, et al., Scientific Reports, 13, 13913, 2023, listed above). ODN (oligodeoxynucleotide; oligo DNA) for adapters to be activated with VTopoI is described in detail in Table 1. Two types of combinations were used for ODN. The first combination used was a set without bisulfite treatment (for native) including PEA1-3C2T-Ext, 1A3G1A-anti-PEA1, and Anti-Ext-1, and the second combination used was a set adapted for bisulfite treatment including PEA1-1T-3C2T-Ext-met, 1A3G1A-anti-PEA1, and Anti-Ext-1.

1 ml of a solution containing 10 µM ODN set for native or bisulfite and 1 × Oligo Hyb (10 mM Tris-HCl, pH 7.5, and 160 mM NaCl) was prepared, incubated at 95°C for 5 minutes and at 55°C for 10 minutes, and then maintained at 30°C. Next, the reaction solution was supplemented with 1 mL of 5 × TOPO activation buffer, 500 µL of 1 mg/mL BSA, 100 µL of 100 mM ATP, and 100 µL of 1 mg/ml T4 PNK and volume-adjusted to 5 mL with water. After the reaction solution was equilibrated at 30°C for 10 minutes, 50 nmol VTopoI was added to the reaction solution, and the resultant was further incubated at 30°C for 1 hour. The formed VOC was purified using a heparin affinity purification column as follows.

All chromatography purification was carried out by manual operation using a 5 mL Luer-Lock syringe (Terumo Corp., Tokyo, Japan) and a syringe pump (YSP101, YMC Co., Ltd., Kyoto, Japan) in combination. First, a 1 mL HiTrap Heparin HP column (Cytiva) was equilibrated with 5 mL of Buffer 300. Next, the reaction product of VTopoI was loaded to the column, and the column was further washed with 5 mL of Buffer 300. Finally, VOC was eluted with 5 mL of Buffer 400. In this respect, the flow rate was constantly kept at 2 mL/min. Then, this eluate was transferred to Amicon-4 30 kDa device, followed by buffer replacement and DNA concentration adjustment using Buffer 1000. The final concentration of VOC was measured using Qubit dsDNA BR Kit (Thermo Fisher Scientific Inc.) and adjusted to 150 ng/µL.

### <Specific contents of present Example>

RNA ligase was screened for with the aim of identifying a new enzyme with stronger single-stranded DNA ligation activity without the attachment of some adenylic acids (i.e., ribotail) to the 3' end of target single-stranded DNA. First, the amino acid sequence database of NCBI (https://www.ncbi.nlm.nih.gov/) was searched with TS2126 RNA ligase as a seed to identify many homologous proteins. These proteins involved T4 RNA ligase (see Non Patent Literatures 10 and 11 listed above), RM378 RNA ligase (see Non Patent Literature 12 listed above), and Mth RNA ligase (see Non Patent Literature 13 listed above), and other proteins (Figure 1A). A phylogenetic tree drawn using their amino acid sequences revealed that the proteins known to have single-stranded DNA ligation activity were relatively distant from the TS2126 RNA ligase. On the other hand, eight proteins (specifically, eight proteins registered under GenBank Accession Nos: AYJ73970, WP _119360902, WP_110532308, WP_126206484, WP_172959156, WP_038069792, WP_081892735, and RTI02525) exhibited higher homology to the TS2126 RNA ligase (Figure 1A). These eight proteins have been identified in various genome sequencing projects with different purposes, and as a matter of course, their single-stranded DNA ligation activity has not yet been examined. Hence, the present inventor examined the single-stranded DNA ligation activity of these proteins. However, of the eight proteins, two pairs (specifically, a pair of WP_172959156 and RTI02525 and a pair of WP_081892735 and WP_038069792) had substantially identical amino acid sequences and structurally differed only in N-terminal extension (Figure 2). Therefore, only two (RTI02525 and WP_081892735) out of the four proteins in these two pairs were examined because of their N-terminal extension.

First, genes encoding the selected enzyme proteins were artificially synthesized, cloned into expression vectors, and the encoded proteins were purified (Figure 3). Next, both the TACS ligation activity (with ribotail) and single-stranded DNA ligation activity (without ribotail) of these proteins were confirmed (Figure 1B). As a result, three out of the six proteins were found to exhibit strong TACS ligation activity (Figure 1C). Two of the proteins exhibited stronger single-stranded DNA ligation activity than that of the TS2126 RNA ligase (Figure 1C). Particularly, AYJ73970 was further examined because AYJ73970 had better single-stranded DNA ligation activity (Figure 1C). The enzyme protein of WP_119360902 is capable of exhibiting results and effects equivalent or similar to various results and effects obtained about the enzyme protein of AYJ73970 given below, and is thus considered useful.

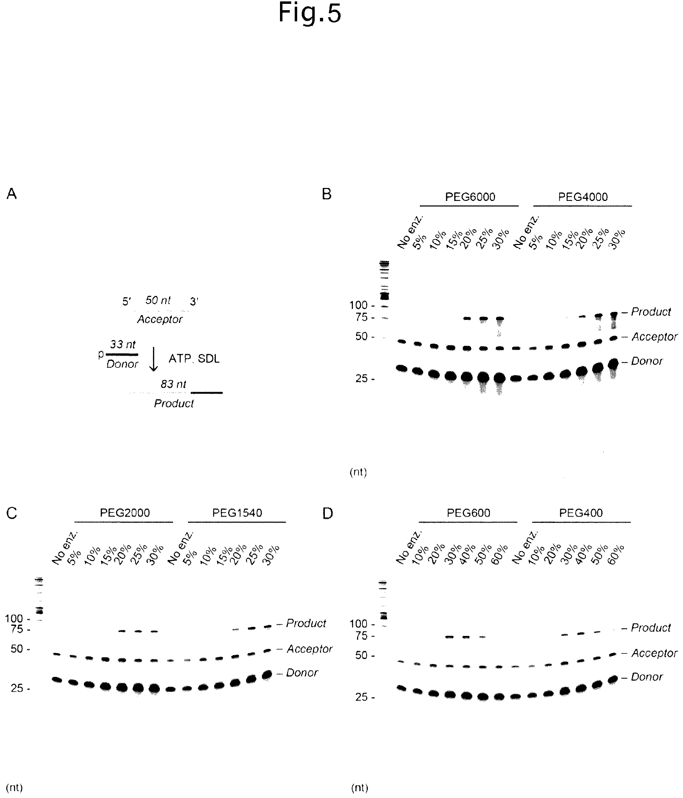

AYJ73970 had single-stranded DNA ligation activity at 20 to 49°C with the optimum temperature of 37°C (Figure 4). The coexistence of polyethylene glycol (PEG) with varying molecular weights was required for the single-stranded DNA ligation activity (Figure 5). pH 7.5 to 9.5 was suitable for the reaction solution, and the activity was confirmed in both Tris-based and TAPS-based buffers (Figure 6). AYJ73970 lost its activity in coexistence with ammonium chloride on the order of 10 mM (Figure 7), whereas this enzyme was active in the presence of 50 mM sodium chloride or potassium chloride, albeit with a preference for lower concentrations (Figure 7). The optimum magnesium concentration was 7.5 to 12.5 mM (Figure 8). Stronger single-stranded DNA ligation activity was confirmed for a donor and an acceptor of 16 or more and 30 or more nucleotides, respectively (Figure 9). While AYJ73970 exhibited equivalent activity against both DNA and RNA acceptors, the enzyme tended to prefer DNA over RNA as the donor (Figure 10). AYJ73970 was capable of ligating a single-stranded DNA adapter to double-stranded DNA, albeit with significantly lower efficiency compared with ligation to single-stranded DNA (Figure 11). AYJ73970 was identified in the genome sequence of the bacteriophage Thermus phage phiLo (i.e., this enzyme should be referred to as Tph ssDNA ligase). In the present Example, this enzyme (AYJ73970) is referred to as "SDL" by taking the initials of the English word "single-stranded DNA ligase".

SDL is the first-ever ligase having strong ligation activity against single-stranded DNA and can therefore be expected to be used in various applications. Hence, SDL was used in some of representative applications. The present inventor previously established the protocol of sequencing library preparation from single-stranded DNA on the basis of TACS ligation. In this method, in order to suppress adapter dimer formation, the first adapter ligation was performed for target single-stranded DNA, and DNA replication beginning at the adapter was performed to form double strands, followed by the second adapter ligation reaction using vaccinia virus topoisomerase I (TOPO) finally (TACS-TOPO method, Figure 12A). Use of this TACS-TOPO method realized a highly sensitive analysis of short single-stranded DNA in the cell-free fraction of human blood. Furthermore, the TACS-TOPO method was able to increase yields in library preparation from ancient human bones by at least an order of magnitude. Hence, the present inventor attempted to replace the TACS ligation step of this TACS-TOPO method with a step using SDL and carried out this method (SDL-TOPO method) (Figure 12).

Use of the SDL-TOPO method achieved library preparation from single-stranded DNA with efficiency equivalent to the TACS-TOPO method, while further advantages of the SDL-TOPO method were also found. For example, RNA is sensitive to alkaline conditions. Bisulfite treatment cannot be applied to sequencing libraries involving ribotail prepared by the TACS-TOPO method because desulfonation treatment with an alkali is an indispensable operation for the bisulfite treatment. On the other hand, libraries prepared by the SDL-TOPO method are RNA-free, and bisulfite treatment can therefore be applied to the libraries prepared by the SDL-TOPO method. In short, the libraries prepared by the SDL-TOPO method can be used to examine the 5-methylation status of cytosines in the insert DNA (Figure 12). As a result of actually applying bisulfite treatment (which was carried out using EZ DNA Methylation Gold Kit from Zymo Research Corp. in accordance with manufacture's instruction; the same holds true for the description below) to the libraries prepared by the SDL-TOPO method, almost all cytosines were eliminated from the reads, while the obtained distribution of read lengths was confirmed to not vary depending on the presence or absence of bisulfite treatment. These results mean that use of SDL-TOPO realizes highly sensitive methylome analysis on cell-free DNA in blood (Figure 12).

The effectiveness of SDL was also confirmed in sequencing library preparation for the quantification of genome-wide DNA methylation levels, i.e., methylome analysis. The present inventor has recently reported the tPBAT method, which is a library preparation method for whole-genome bisulfite sequencing using TACS ligation (see Non Patent Literature 7 listed above). In the tPBAT method, the first adapter sequence is introduced by random priming with bisulfite-treated DNA as a template. Next, TACS ligation is applied to the first-strand DNA so that the second adapter is ligated thereto to complete library preparation. The TACS ligation of this tPBAT method can also be substituted with SDL (sPBAT method). As a result, use of the sPBAT method produced libraries two to three times more than those obtained by tPBAT (Figure 13). The quality of reads obtained in sPBAT was similar to that of tPBAT, with the same results on quantitation of DNA methylation levels (Figure 13). The operation of sPBAT is more simplified than that of tPBAT, and library yields are also higher in sPBAT. Thus, it can be said that this approach is capable of producing more practical and highly sensitive methylome data (Figure 13). In this context, the details of the sPBAT protocol are as described in the above description of Figure 13 (section of Brief Description of Drawings). Hereinafter, the comparison between tPBAT and sPBAT will be described.

### <Comparison between tPBAT and sPBAT>

To 100 ng of genomic DNA purified from an IMR-90 cell line (JCRB9054, Japanese Collection of Research Bioresources Cell Bank) or derived from human female peripheral blood (Promega Corp.), 1 ng of unmethylated lambda DNA (Promega Corp.) was added, and the resultant was subjected to bisulfite treatment. tPBAT was carried out by the previously reported approach (see Miura F. et al., 2019 (Non Patent Literature 7)), and sPBAT was carried out by the protocol mentioned above. Equimolar amounts of tPBAT and sPBAT libraries labeled with different indexes were mixed with PhiX control (Illumina, Inc., 20% with respect to each library) and sequenced (Macrogen Japan Corp., Tokyo, Japan) in the 150 × 2 paired-end mode using HiSeq X Ten System (Illumina, Inc.).

The applications of SDL were not limited to methylome analysis. SDL can also be used in, for example, the amplification of cDNA. For this purpose, first, cDNA is synthesized using an oligo dT primer 5'-terminally tailed with an adapter sequence for amplification. Next, the 3' end of the synthesized cDNA is tailed with another adapter using SDL. The cDNA is thus ligated at both ends with the different adapters after reaction of SDL and thereby becomes capable of being amplified by PCR (Figure 14). The amplified cDNA was actually able to be sequenced with a long-read sequencer. Many reads covered the whole genes, and practical cDNA analysis was able to be achieved (Figure 14).

In the present Example, SDL (AYJ73970) was identified as an enzyme having better single-stranded DNA ligation activity than that of known enzymes. The use of SDL in four sequencing library preparations was demonstrated as application examples. However, the applications of SDL are not limited to such sequencing library preparations. SDL would be useful for, for example, combinatorial indexing. More convenient cell labeling with indexes may be carried out by SDL which realizes operation of ligating a mixed solution of 5'-terminally phosphorylated chemically synthesized DNA to target DNA. Furthermore, SDL is also likely to contribute to artificial gene library synthesis. SDL is also considered suitable for, for example, randomly mixed amino acid codons and module shuffling of particular amino acid sequences. SDL is capable of contributing to the realization of many other applications.

The enzyme protein of WP_119360902 which was found to have stronger single-stranded DNA ligation activity than that of the TS2126 RNA ligase, as in SDL (AYJ73970), is available in the same or similar application examples (four sequencing library preparations) as those of SDL as mentioned above and is also considered available in various other possible applications.

### Industrial Applicability

The present invention can provide a composition for ligating single-stranded DNA as a novel enzyme composition having efficient single-stranded DNA ligation activity, a single-stranded DNA ligation agent, a kir for ligating single-stranded DNA, and the like. Use of the composition for ligating single-stranded DNA or the like according to the present invention enables single-stranded DNAs as a donor and an acceptor to be ligated without ribotailing and for example, can achieve highly efficient sequencing library preparation. In this respect, the composition for ligating single-stranded DNA or the like according to the present invention is useful.

### Free Text of Sequence Listing

SEQ ID NOs: 1, 3, 5 to 12, 14 to 16 and 18 to 31: Synthetic DNA
SEQ ID NOs: 13 and 17: Synthetic RNA

## Claims

1. A composition for ligating single-stranded DNA, used for ligating at least one single-stranded DNA as an acceptor to at least one single-stranded DNA as a donor, the composition comprising
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

2. The composition according to claim 1, wherein the acceptor DNA is 12 or more bases long, and/or the donor DNA is 10 or more bases long.

3. The composition according to claim 1, further comprising PEG.

4. A single-stranded DNA ligation agent, used for ligating at least one single-stranded DNA as an acceptor to at least one single-stranded DNA as a donor, the ligation agent comprising
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

5. The ligation agent according to claim 4, wherein the acceptor DNA is 12 or more bases long, and/or the donor DNA is 10 or more bases long.

6. The ligation agent according to claim 4, further comprising PEG.

7. A kir for ligating single-stranded DNA, used for ligating at least one single-stranded DNA as an acceptor to at least one single-stranded DNA as a donor, the kit comprising
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

8. The kit according to claim 7, wherein the acceptor DNA is 12 or more bases long, and/or the donor DNA is 10 or more bases long.

9. The kit according to claim 7, further comprising PEG.

10. A method for producing ligated single-stranded DNA, comprising bringing at least one single-stranded DNA as an acceptor and at least one single-stranded DNA as a donor into contact with
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity,
to ligate the acceptor DNA to the donor DNA.

11. A method for ligating single-stranded DNA, comprising bringing at least one single-stranded DNA as an acceptor and at least one single-stranded DNA as a donor into contact with
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity,
to ligate the acceptor DNA to the donor DNA.

12. The method according to claim 10 or 11, wherein the acceptor DNA is 12 or more bases long, and/or the donor DNA is 10 or more bases long.

13. Use of
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity
in the method according to claim 10 or 11.

14. (a) A protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity
for use in the method according to claim 10 or 11.

15. A method for preparing a double-stranded DNA library from a single-stranded DNA fragment, the preparation method comprising the steps of:
(A) ligating a 5'-terminally phosphorylated single-stranded adapter sequence to the target single-stranded DNA fragment;
(B) performing DNA replication beginning at the adapter of the ligated sequence to form double-stranded DNA;
(C) ligating a topoisomerase-complexed double-stranded adapter sequence to ends tailed with no adapter sequence of the double-stranded DNA; and
(D) using the ligated double-stranded DNA as a template in PCR to obtain a library comprising a plurality of the double-stranded DNA, wherein
the ligation in the step (A) is performed by a ligation method using
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

16. A method for preparing a double-stranded DNA library from a single-stranded DNA fragment, the preparation method comprising the steps of:
(i) ligating a 5'-terminally phosphorylated single-stranded adapter sequence A1 to the 3' end of a single-stranded DNA fragment dephosphorylated at both ends to form adapter-tailed single-stranded DNA;
(ii) annealing a single-stranded adapter sequence A2 to the adapter sequence A1 moiety contained in the adapter-tailed single-stranded DNA, followed by extension from the 3' end of the adapter sequence A2 with DNA polymerase to form adapter-tailed double-stranded DNA;
(iii) reacting a double-stranded adapter sequence B1 with topoisomerase to form a topoisomerase-complexed double-stranded adapter sequence B2, wherein the double-stranded adapter sequence B1 is phosphorylated at only one 5' end and has a topoisomerase recognition sequence in a region containing a 3' end complementary to this phosphorylated 5' end, and wherein the topoisomerase-complexed double-stranded adapter sequence B2 has the topoisomerase conjugated with the 3' end of the topoisomerase recognition sequence;
(iv) ligating the topoisomerase-complexed double-stranded adapter sequence B2 to ends tailed with no adapter sequence of the adapter-tailed double-stranded DNA to form both-terminally adapter-tailed double-stranded DNA; and
(v) using the both-terminally adapter-tailed double-stranded DNA as a template in PCR to obtain a library comprising a plurality of the double-stranded DNA, wherein
the ligation and the formation in the step (i) are performed by a ligation method using
(a) a protein comprising an amino acid sequence represented by SEQ ID NO: 2 or 4, and/or
(b) a protein which comprises an amino acid sequence having 70% or higher identity to the amino acid sequence represented by SEQ ID NO: 2 or 4 and has single-stranded DNA ligation activity.

17. A double-stranded DNA library obtainable by the preparation method according to claim 15 or 16.
